(19) Europäisches Patentamt
European Patent Office
Office européen des brevets

(11) EP 3 789 757 B1

# (12) EUROPEAN PATENT SPECIFICATION

(45) Date of publication and mention of the grant of the patent:
**05.11.2025 Bulletin 2025/45**

(21) Application number: **19791629.9**

(22) Date of filing: **26.04.2019**

(51) International Patent Classification (IPC):
***G01N 21/64*** *(2006.01)* ***G01N 33/48*** *(2006.01)*
***G01N 15/14*** *(2024.01)* ***G01N 1/30*** *(2006.01)*
***G01N 1/31*** *(2006.01)* ***G01N 33/49*** *(2006.01)*
***G01N 35/10*** *(2006.01)* ***G01N 15/10*** *(2024.01)*
***G01N 35/00*** *(2006.01)*

(52) Cooperative Patent Classification (CPC):
**G01N 33/49; G01N 15/1459; G01N 21/64; G01N 33/48;** G01N 1/30; G01N 1/31; G01N 35/1095; G01N 2015/1006; G01N 2015/1402; G01N 2035/00356

(86) International application number:
**PCT/CN2019/084645**

(87) International publication number:
**WO 2019/206297 (31.10.2019 Gazette 2019/44)**

## (54) BLOOD ANALYZER AND ANALYSIS METHOD

BLUTANALYSEGERÄT UND ANALYSEVERFAHREN

ANALYSEUR DE SANG ET PROCÉDÉ D'ANALYSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2018 CN 201810402417**

(43) Date of publication of application:
**10.03.2021 Bulletin 2021/10**

(73) Proprietor: **Shenzhen Mindray Bio-Medical Electronics Co., Ltd.**
**Shenzhen, Guangdong 518057 (CN)**

(72) Inventors:
- **YE, Bo**
  **Shenzhen, Guangdong 518057 (CN)**
- **QI, Huan**
  **Shenzhen, Guangdong 518057 (CN)**
- **ZHENG, Wenbo**
  **Shenzhen, Guangdong 518057 (CN)**
- **YE, Yi**
  **Shenzhen, Guangdong 518057 (CN)**

(74) Representative: **KIPA AB**
**Drottninggatan 11**
**252 21 Helsingborg (SE)**

(56) References cited:
**EP-A1- 0 774 113** **EP-A1- 1 574 839**
**CN-A- 1 149 337** **CN-A- 101 236 158**
**CN-A- 101 236 195** **CN-A- 101 236 195**
**CN-A- 104 749 144** **CN-A- 104 749 144**
**JP-A- 2011 237 462** **US-B1- 6 197 593**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### TECHNICAL FIELD

The present invention relates to a blood analyzer and an analysis method thereof.

### BACKGROUD ART

Blood cells are divided into three classes, namely red blood cells (RBC), white blood cells (WBC) and platelets (PLT). When blood routine examination is conducted, it is often needed to detect the classification and quantity of white blood cells, and the quantity of red blood cells and the quantity of platelets.

For detection of white blood cells in blood, it is generally needed to hemolyze blood cells and then detect the white blood cells using flow cytometry or impedance method. For example, a detection process for detecting white blood cells using flow cytometry is as follows: a blood sample is hemolyzed so that red blood cells are lysed into fragments, scattered light of cell particles in the hemolyzed sample is detected through flow cytometry, and white blood cells are differentiated according to scattered light intensity. It is difficult to detect red blood cells during the detection process because they are lysed into small-volume fragments, and the interference of red blood cells on white blood cells can be reduced when identifying the white blood cells.

For counting of red blood cells and platelets, most current medical particle analyzers (including blood cell analyzers) employ aperture impedance principle (hereinafter referred to as electrical impedance method) for detection. The essential basis of the electrical impedance method is the Coulter principle, the usual detection process thereof is as follows: when particles suspended in an electrolyte pass through a detection aperture along with the electrolyte, the equivalent resistance across the detection aperture changes, and the constant current source across the aperture causes change in the voltage across the detection aperture. The voltage change is collected through a circuit system to generate a voltage pulse waveform. The height of the pulse waveform reflects cell volume size and characterizes particle volume information. Therefore, on this base, the analyzers can provide volume distribution of the detected particles, that is, provide a volume distribution histogram of the detected particles. By analyzing the volume distribution histogram of the cells, classification, count and other operations of cells can be performed, wherein large-volume cells are identified as red blood cells, and small-volume cells are identified as platelets.

However, the electrical impedance method is not capable of differentiating mature red blood cells and reticulocytes (RET) in the result of identified red blood cells, let alone reticulocytes having different maturation degrees. The current researches suggest that reticulocytes, as an important stage during the maturation of red blood cells, should also be emphasized.

Reticulocytes (RET) are cells at the stage between orthochromatic normoblasts and mature red blood cells, and are slightly larger than mature red blood cells. Reticulocytes are cells at the stage in which erythroblasts have just lost their nuclei. Reticulocytes are still red blood cells that are not fully matured, and ribosomes, ribonucleic acids and other basophilic substances are still remained in cytoplasm of reticulocytes. After being stained in vivo by brilliant cresyl blue or new methylene blue, blue or blue-green dendrites or even reticulations can be seen in cytoplasm, so they are called reticulocytes.

In recent years, instruments for RET count using flow cytometry have come out one after another, methods thereof are as follows: a dye is used to stain RET, and a sheath flow technology and a radio frequency technology are used for detection. For example, Chinese patent CN 97110727 has disclosed a flow cytometry measurement method, which can measure reticulocytes by measuring forward scattered light intensity and fluorescent light intensity. This detection solution has the advantages of good repeatability, high accuracy, time saving and the like, and thus is being used by more and more hospitals.

EP1574839 A1 has disclosed a process of analyzing reticulocytes (RET) and mature erythrocytes using the optical detection unit. When analyzing reticulocytes (RET) and mature erythrocytes, the sample preparation unit dilutes a blood sample, and stains the diluted blood sample using a predetermined stain. Consequently, the prepared blood sample is supplied to the optical detection unit. Then, the optical detection unit irradiates the prepared blood sample with light, and outputs electrical signals representing the forward scattered light intensity, lateral scattered light intensity, and lateral fluorescent light intensity. The body side controller analyzes the reticulocytes and mature erythrocytes using the data representing the forward scattered light intensity and lateral fluorescent light intensity.

CN101236195A and JP2011237462A have disclosed a specimen analyzer capable of analyzing a blood sample and a body fluid, in which a predetermined amount of sample collected by the sampling valve, a predetermined amount of dilution agent, and a predetermined amount of stain solution are supplied to the reaction chamber by a dosage pump which is not shown in the drawing, the sample and reagents are then mixed to prepare a measurement sample for measuring reticulocytes (RET).

EP0774113A1 has disclosed a reagent system for reticulocyte detection. The reagent system comprises an

unsymmetrical cyanine dye capable of staining reticulocytes, a buffer, a pH, an osmolarity and a non-ionic surfactant. Another ingredient of the reagent system is a non-ionic surfactant. Such surfactant(s) should not, however, precipitate or react with the cyanine dyes or lyse RBCs, even at the low concentrations. Yet another ingredient of the reagent system is a mono-, or di-, valent alkali salt to adjust the osmolarity of the reagent from about 230 mOsm/L to about 340 mOsm/L to prevent the lysis of red cells, including the reticulocytes, or the white cells.

CN104749144A provides a reagent for detecting blood cells, wherein the reagent includes a fluorescent dye, a spheroidization component and an organic alcohol. The fluorescent dye is cell-permeable and is capable of specifically staining nucleic acid substances in cells. The spheroidization component is capable of spheroidizing red blood cells with the red blood cell membrane intact and without damaging the internal structure of white blood cells. The organic alcohol is capable of increasing cell permeability and assisting the fluorescent dye enter into cells. By using the reagent, cells, such as reticulocytes, mature white blood cells and immature white blood cells can be stained without destroying cell membrane of blood cells.

However, it is needed to design a separate detection channel in a cell analyzer if flow cytometry is used to detect reticulocytes, thereby increasing detection costs of instruments and reagents.

## SUMMARY OF THE INVENTION

The present invention provides a new detection solution for reticulocytes.

According to a first aspect, an embodiment provides a blood analyzer according to claim 1.

According to a second aspect, an embodiment provides a blood analysis method according to claim 8.

In the embodiments of the present invention, the test blood sample subjected to hemolysis is detected in a white blood cell measurement channel, and reticulocyte particles are thus identified utilizing data of blood ghost region, thereby realizing differentiation of reticulocytes and large platelets and finer classification and count of reticulocytes without separately designing an optical detection channel in blood analyzer.

## BRIEF DESCRIPTION OF THE DRAWINGS

Fig. 1 is a scattergram of a reticulocyte channel (RET channel) of Mindray BC-6800.

Fig. 2 is a structural diagram of relevant parts of a blood analyzer in an embodiment;

Fig. 3a is a side scattered light-fluorescent light scattergram;

Fig. 3b is a forward scattered light-fluorescent light scattergram;

Fig. 3c is an enlarged view of a blood ghost region;

Fig. 3d is a diagram for identifying nucleated red blood cells while identifying reticulocyte particles through the forward scattered light-fluorescent light scattergram;

Fig. 4a is a flow chart for identifying reticulocyte particles in an embodiment;

Fig. 4b is a flow chart for identifying large platelets and reticulocyte particles in an embodiment;

Fig. 5 is a diagram showing reticulocyte populations in an embodiment;

Fig. 6 is a volume distribution histogram of large platelets in an embodiment;

Fig. 7a-7d are comparison diagrams of large platelet count information obtained by adopting the solution of the present invention and BC-6800 blood cell analyzer;

Fig. 7e-7h are comparison diagrams of reticulocyte count information obtained by the solution of the present invention and BC-6800 blood cell analyzer.

## DETAILED DESCRIPTION OF EMBODIMENTS

Next, the present invention will be further described in detail through embodiments in combination with drawings. Similar elements in different embodiments adopt relevant similar reference numerals. In the following embodiments, description of many details is to better understand the present application. However, those skilled in the art can realize without efforts that a part of features can be omitted in different cases, or can be replaced with other elements, materials and methods. In some cases, some operations related to the present application are not shown or described in the Specification, which is to avoid the ambiguousness of the core features of the present application due to excessive detailed descriptions. However, for those skilled in the art, it is not necessary to describe these relevant operations in detail, they can completely know relevant operations according to description of the Specification and ordinary technical knowledge in the art.

In addition, features, operations or characteristics described in the Specification may be combined in any proper manners to obtain various embodiments. Meanwhile, various steps and actions in the description of methods may also be subjected to sequence regulation or adjustment according to manners that are obvious for those skilled in the art. Thus, various sequences in the Specification and drawings are only for clearly describing a certain embodiment, but are not necessary sequences, unless otherwise stated that a sequence must be followed.

Numerals themselves of components herein, for example "first" and "second", are only used for distinguishing among described objects, and have no any sequence or technology meaning. The terms "connection" and "couple" in the present application, if no specifically stated, all include direct and indirect connection (couple).

In blood cell analyzers, both of white cell classification and reticulocyte detection adopt flow cytometry, but they cannot be performed in a common measurement channel. The reason is that: in white blood cell measurement channel, in order to reduce interference of red blood cells on white blood cells, hemolytic treatment of blood samples is designed to lyse red blood cells into smaller fragments under the effect of hemolytic agents, so as not to affect white blood cell measurement. However, when reticulocytes are detected, it is not expected to destroy reticulocytes in theory. Since hemolytic agents are also capable of lysing reticulocytes, hemolytic treatment of blood samples is not designed in existing reticulocyte measurement channel which adopts flow cytometry.

The contribution of the present invention is that, a solution of detecting reticulocytes and/or large platelets by using white blood cell measurement channel has been found. This solution can detect quantity or other information of large platelets as well as quantity or other information of reticulocytes, for example more finely classify and count reticulocytes. It is crucial in the present invention to discover new use of data of blood ghost region.

Referring to Fig. 2, Fig. 2 shows a structural diagram of relevant parts of a blood analyzer according to a specific embodiment of the present invention, including a test sample preparation apparatus, a measurement apparatus 22, a data processor 25 and a display 26.

The test sample preparation apparatus includes a sampling device (not shown) and a reaction cell 21. The sampling device may be a sampling needle or a sampling valve which discharges sucked test blood sample or reagent to the reaction cell 21 through suction and discharge actions. Separate sampling needles or sampling valves may be respectively used for sample and reagent, or a common sampling needle or sampling valve may be used for sample and reagent. The sampling device may also include a sampling needle which is used for discharging sucked test blood sample to the reaction cell 21 through suction and discharge actions, and a hydraulic pipeline which is used for injecting reagent into the reaction cell 21 through positive pressure or negative pressure. In an embodiment of the present invention, the reagent at least includes a fluorescence staining agent and a hemolytic agent. The reaction cell 21 provides a place for mixing and incubation of the test blood sample and the fluorescence staining agent as well as the hemolytic agent. When mixing the sample with the fluorescence staining agent and the hemolytic agent, both or three of them are simultaneously added, or added in sequence. After the sample is mixed with the fluorescence staining agent and the hemolytic agent, red blood cells in the test blood sample are lysed under the effect of the hemolytic agent, and cells containing nucleic acid are stained by the fluorescence staining agent. A test sample for measurement are prepared after mixing and incubation of the test blood sample, the fluorescence staining agent and the hemolytic agent.

The measurement apparatus 22 includes a light source 22a, a flow chamber 22c and optical detection devices 22f, 22k and 22g. The flow chamber 22c provides an area for cell particles in the test sample to pass through one by one and to be irradiated with light. Generally, the flow chamber 22c is communicated with the reaction cell 21 through a pipeline 27. When the test sample for measurement is prepared, the test sample reaches an inlet of the flow chamber 22c through the pipeline 27, and then cells particles in the test sample pass through the flow chamber 22c one by one in a state of being surrounded by a sheath fluid. Light emitted by the light source 22a is converged to an optical detection area of the flow chamber 22c after passing through a light collimating lens 22b. When the cell particles pass through the optical detection area of the flow chamber 22c, the light beam is irradiated on the cell particles. Different scattered lights are generated by different types of cells. In addition, fluorescent light can be also generated by cells stained by the fluorescence staining agent. The optical detection devices collect light at two scattering angles and fluorescent light. First angle scattered light is scattered light whose included angle with optical axis is relatively small, for example, the first angle scattered light includes

scattered light whose included angle with optical axis is within a range of 1-6°, and second angle scattered light is scattered light whose included angle with optical axis is relatively large, for example, the second angle scattered light includes scattered light whose included angle with a direction perpendicular to optical axis is within a range of 1-6°. The first angle scattered light signal is used for reflecting volume size information of cell particles, and the second angle scattered light signal is used for reflecting complexity information of cell particles. Generally, the first angle scattered light is forward scattered light, and the second angle scattered light is side scattered light. The forward scattered light refers to scattered light collected in the direct direction of light beam (i.e. optical axis direction), and the side scattered light refers to scattered light collected in a direction perpendicular to optical axis. It should be understood that in other embodiments, those skilled in the art can also set the first angle and the second angle to be within other angle ranges as required, as long as they respectively reflect volume size and complexity of cells. In an embodiment of the present invention, only the first angle scattered light signal and the fluorescent light signal may be detected, or only the first angle scattered light signal may be detected. The optical detection devices 22f, 22k and 22g may be for example photoelectric sensors which sense light intensity and convert optical signals into electrical signals to be output, and the output electrical signals are output to the data processor 25 after being amplified by an amplifier 23.

In the embodiment as shown in Fig. 2, forward scattered light generated by cells is irradiated on the light detector 22f after passing through a converging lens 22d and a microplate 22e. The light detector 22f senses light intensity and converts light signals into electrical signals to be output, which are called forward scattered light signals. Side light generated by cells is split into two beams of side scattered light and fluorescent light after light splitting via a beam splitter 22h, wherein the side scattered light is incident to the light detector 22g, and the light detector 22g senses light intensity and converts light signals into electrical signals to be output, which are called side scattered light signals; the fluorescent light is irradiated on the light detector 22k after passing through a converging lens 22i and a filter 22j, and the light detector 22k senses fluorescent light intensity and converts light signals into electrical signals to be output, which are called fluorescent light signals. The forward scattered light signals, the fluorescent light signals and the side scattered light signals are transmitted to the data processor 25 after being amplified via amplifiers 23a, 23b and 23c respectively.

The data processor 25 is used for operating data to obtain required results. For example, a two-dimensional scattergram may be generated according to various collected light signals of the cell particles, or a three-dimensional scattergram may be generated, and white blood cell analysis or other analysis may be performed on the scattergram according to gating method. The data processor 25 may also visualize intermediate operation results or final operation results, and then the visualized results are displayed on a display 27.

In an embodiment of the present invention, the data processor 25 identifies large platelets and/or reticulocytes according to the first angle scattered light signals and the fluorescent light signals of the cell particles, or identifies large platelets and/or reticulocytes according to first angle scattered light signals of the cell particles, and then large platelet information and/or reticulocyte information is/are obtained.

In research process, the inventors detected forward scattered light signals (FSC), side scattered light signals (SSC) and fluorescent light signals (FL) of cell particles using white blood cell channel, and a side scattered light-fluorescent light scattergram and a forward scattered light-fluorescent light scattergram are generated, wherein Fig. 3a shows the side scattered light-fluorescent light scattergram, and Fig. 3b shows the forward scattered light-fluorescent light scattergram. In the scattergrams of Fig. 3a and Fig. 3b, cell particles can be basically divided into two large particle regions, namely, a white blood cell region 31a, 31b with large fluorescent light signals and a blood ghost region 32a, 32b with small fluorescent light signals. The blood ghost region is also considered as a non-white blood cell region, which is residue after red blood cells and platelets in blood are hemolyzed and commonly referred to as blood ghost. In various types of analysis, data of the blood ghost region is often ignored as abandoned data. The inventors found that in the forward scattered light-fluorescent light scattergram, the blood ghost region 32b is of a special shape, and by enlarging the blood ghost region 32b in Fig. 3c, the blood ghost region 32b exhibits two branches, namely a first branch 33 extending in the forward scattered light direction and a second branch 34 extending in the fluorescent light direction. After theoretical analysis and experiments, the inventors are of the opinion that the two branches can be utilized to differentiate large platelets and reticulocytes.

The exiting research results have proved that although original volumes of red blood cells are larger than that of platelets, red blood cells are lysed into small-volume fragments after hemolysis with a hemolytic agent. For small platelets (such as platelets with a volume of less than 10 fL), due to small volumes, the platelets are further reduced in volume after being treated with the hemolytic agent. For these small-volume particles, on the one hand, they are limited by detection low limit of systems; on the other hand, small platelets and red blood cell fragments are similar in volume, so it is difficult to accurately differentiate between them.

Upon researches, the inventors have realized that after blood is hemolyzed with a hemolytic agent, mature red blood cells are lysed to form small-volume fragments which are not easily identified due to their small volumes. However, cytoplasm of reticulocytes contains basophilic dot-like or even reticulocyte-like particles. These particles are difficult to be lysed, so hemolyzed reticulocytes will be slightly larger than lysed mature red blood cells in volume. At the same time, because these particles contain nucleic acids, after staining, fluorescent light information thereof will be greater than that of

platelets, and the more the basophilic particles are, the stronger the fluorescent light is. Furthermore, the inventors believe that after blood sample is hemolyzed, the volumes of platelets are decreased, but the relative sizes are still kept unchanged, that is, the volumes of platelets that are originally large in volume after hemolysis are still large, the platelets that are large in volume can be detected in hemolytic channel through flow cytometry. Based on the above analysis, it can be concluded that the first branch 33 extending in the forward scattered light direction is large platelets, while the second branch 34 extending in the fluorescent light direction is reticulocyte particles. The reticulocyte particles may be basophilic particles which are wrapped by cell membranes and treated with hemolytic agent, or basophilic particles which are released after cell membranes are destroyed. Because the above particles contain nucleic acids, their fluorescent light information will also be larger than that of ordinary mature red blood cells, and the more the basophilic particles are, the stronger the fluorescent light is. The reticulocyte particles herein refer to particles characterizing reticulocytes. Via researches, the inventors have found that the quantity of these particles is related to the quantity of reticulocytes in sample. A mixture of mature red blood cell fragments and small platelets is located in the lower left corner region of the blood ghost.

Based on this conclusion, it is envisaged that an appropriate region, such as a second region 35, is set on the first branch 33 by gating, so that particles in this region are large platelets. The second region 35 refers to a region in the blood ghost region where the fluorescent light signal intensities are relatively small and the first angle scattered light signal intensities are relatively large. In practical operation, the position and size of the second region 35 may be set based on a region where large platelets should or may occur as recognized according to theory and/or experience of those skilled in the art, that is, in the second region 35, cell particles can be identified as large platelets; furthermore, toward the right along the forward scattered light FSC, the stronger the forward scattered light signals are, the larger the volumes of platelets are. Similarly, an appropriate region, such as a first region 36, is also set on the second branch 34 by gating, so that particles in this region are reticulocytes. The first region 36 refers to a region in the blood ghost region where the fluorescent light signal intensities are relatively large and the first angle scattered light signal intensities are relatively small. The position and size of the first region 36 may also be set based on a region where reticulocytes should or may occur as recognized according to theory and/or experience of those skilled in the art, that is, in the first region 36, cell particles can be identified as reticulocytes; furthermore, along the upward direction of the fluorescent light FSC, the stronger the fluorescent light signals are, the more the basophilic particles in the reticulocytes are, and the more immature the reticulocytes are.

Next, various applications will be described through specific embodiments.

In an embodiment, description is made by taking analysis of forward scattered light signals and fluorescent light signals of cell particles as an example. A treatment flow for analyzing a test blood sample is shown in Fig. 4a, including the following steps:

Step 100, collecting a test blood sample. The test blood sample is quantitatively collected through a sampling needle or a sampling valve, and a certain amount of the test blood sample is injected into a reaction cell.

Step 101, performing hemolytic reaction on the blood sample with a hemolytic agent. The hemolytic agent is added to the reaction cell so that the hemolytic agent is mixed with the blood sample. The hemolytic agent is a reagent that allows red blood cells in the blood to be hemolyzed. Thus, red blood cells are lysed under the effect of the hemolytic agent, and red blood cells are reduced in volume to become cell fragments.

Step 102, performing fluorescence staining on the test blood sample with a fluorescence staining agent. The fluorescence staining agent is added into the reaction cell so that the fluorescence staining agent is mixed with the blood sample. The fluorescence staining agent is a reagent that is used for staining nucleic acid. White blood cells are stained because they contain nucleic acids.

**In** another embodiment, the hemolytic agent and the fluorescence staining agent are simultaneously added into the reaction cell 21. **In** addition, other types of reagents may also be added according to detection demand.

Step 103, mixing the test blood sample, the hemolytic agent and the fluorescence staining agent that are respectively added into the reaction cell 21, and incubating them for a certain while to prepare a test sample for measurement.

The fluorescence staining agent herein may be a conventional dye for detecting white blood cells or nucleated red blood cells, for example a dye involved in Chinese Parent Application with the application No. CN200910177186.7 or a dye involved in Chinese Patent Application with the application No. CN200910238927.8. The hemolytic agent may be a conventional hemolytic agent, for example, surfactants involved in these two patents can be prepared into a hemolytic agent.

Step 104, detecting the test sample by flow cytometry. Through fluid path control, a certain amount of the test sample in the reaction cell is flowed to a flow chamber and is mixed with a sheath fluid at inlet of the flow chamber, and cell particles in the test sample are passed through an optical detection area one by one in a state of being surrounded by the sheath fluid. A light beam emitted by a light source is irradiated on the optical detection area, and the irradiated light is scattered by the cell particles, the scattered lights of different types of cell particles are different. Meanwhile, because the cell particles are stained, when the cell particles stained by the fluorescence staining agent are irradiated with light, the cell particle generate fluorescent light whose wavelength is longer than that of the irradiated light. The more the nucleic acids are, the stronger the staining is, and the stronger the emitted fluorescent light is.

Step 105, collecting scattered light and fluorescent light of the cell particles under the irradiation of light, and outputting scattered light signals and fluorescent light signals by an optical detection device according to light intensities of the collected scattered light and fluorescent light. In one embodiment, a light collection system shown in Fig.2 may be used, the scattered light signals includes forward scattered light signals and side scattered light signals, the forward scattered light is collected through a forward scattered light collecting device, and the side scattered light is collected through a side scattered light collecting device, wherein the forward scattered light signals are used for reflecting volume size information of cell particles, and the side scattered light signals are used for reflecting complexity of cell particles.

Step 106, obtaining data characterizing the cell particles. Each cell particle would generate three data, namely, a forward scattered light signal, a side scattered light signal and a fluorescent light signal when passing through the optical detection area, namely, each cell particle is characterized by three data.

These data characterizing the cell particles may be temporarily stored in a buffer, or in a memory, for example, three data of each cell particle are stored as a three-dimensional data set, each data set includes three data, namely forward scattered light information, side scattered light information and fluorescent light information. Multiple data sets of cell particles may form a data matrix, the data processor reads the data of the cell particles from the buffer or the memory for subsequent treatment. Of course, these data characterizing the cell particles may also be directly transmitted to the data processor. The data processor can identify particles according to at least two dimensions of the data sets characterizing the cell particles, for example, in this embodiment, the data processor can identify reticulocytes according to the forward scattered light information and the fluorescent light information, for example the forward scattered light information and the fluorescent light information are respectively compared with corresponding preset standards. According to comparison results, particles falling within the predetermined range are identified as reticulocytes. As another example, particles can also be identified using the following scattergram method.

In some embodiments, if only two-dimensional data is required to identify particles, the light collecting system may also only collect two of the forward scattered light information, the side scattered light information and the fluorescent light information.

Step 108, generating a scattergram based on the data characterizing the cell particles, for example, generating a forward scattered light-fluorescent light scattergram according to the forward scattered light information, the side scattered light information and the fluorescent light information, as shown in Fig. 3b.

Step 113, identifying particles in a first region 36 as reticulocytes.

In further embodiments, when it is needed to further count and/or apply the reticulocytes, the following steps may also be executed.

Step 114, obtaining reticulocyte information according to the identified reticulocytes. The reticulocyte information includes at least one of the followings: reticulocyte marker information, reticulocyte count information (RET), high fluorescent reticulocyte count information, middle fluorescent reticulocyte count information, low fluorescent reticulocyte count information, immature reticulocyte count information and nucleic acid content in reticulocytes.

The reticulocyte count information includes the quantity of reticulocytes (RET#) and/or the ratio of reticulocytes (RET%), wherein particles in the first region may be counted to obtain the quantity of reticulocytes, and the ratio of reticulocytes is a ratio of the quantity of reticulocytes to the quantity of red blood cells in the same measurement sample.

In the first region 36, along the upward direction of the fluorescent light FL, the stronger the fluorescent light signals are, the more the nucleic acids in the reticulocytes are, the more the basophilic particles are, and the more immature the reticulocytes are. The data processor may acquire high fluorescent reticulocyte count information, middle fluorescent reticulocyte count information and low fluorescent reticulocyte count information according to fluorescent light signal distribution of particles in the first region 36. For example, a third region 37, a fourth region 38 and a fifth region 39 may be set in the first region 36 by gating method, as shown in Fig. 5.

High fluorescent reticulocytes exist in the third region 37, the position of which may be set based on a region where juvenile reticulocytes should or may occur as recognized according to theory and/or experience of those skilled in the art. The high fluorescent reticulocyte count information includes a high fluorescent ratio (HFR) and/or a high fluorescent reticulocyte count value (HFR#). Particles in the third region 37 may be counted to obtain the high fluorescent reticulocyte count value HFR#, and the high fluorescent ratio HFR is a ratio of the quantity of high fluorescent reticulocytes to the quantity of reticulocytes in the same measurement sample.

Middle fluorescent reticulocytes exist in the fourth region 38, the position of which may be set based on a region where reticulocytes that are not enough mature should or may occur as recognized according to theory and/or experience of those skilled in the art. The middle fluorescent reticulocyte count information includes a middle fluorescent reticulocyte count value and/or a middle fluorescent ratio (MFR). Particles in the fourth region 38 may be counted to obtain the middle fluorescent reticulocyte count value MFR#, and the middle fluorescent ratio MFR is a ratio of the quantity of middle fluorescent reticulocytes to the quantity of reticulocytes in the same measurement sample.

Low fluorescent reticulocytes exist in the fifth region 39, the position of which may be set based on a region where mature reticulocytes should or may occur as recognized according to theory and/or experience of those skilled in the art. The low fluorescent reticulocyte count information includes a low fluorescent reticulocyte count value and/or a low

fluorescent ratio (LFR). Particles in the fifth region 39 may be counted to obtain the low fluorescent reticulocyte count value LFR#, and the low fluorescent ratio LFR is a ratio of the quantity of low fluorescent reticulocytes to the quantity of reticulocytes in the same measurement sample.

The immature reticulocyte count information includes immature reticulocyte count value and/or immature reticulocyte fraction (IRF). The immature reticulocyte fraction is equal to a sum of MFR and HFR, and the immature reticulocyte count value may be obtained by conversion $IRF\# = RET\# * IRF$. Or the immature reticulocyte count value IRF# may be obtained according to the quantity of high fluorescent reticulocytes and the quantity of middle fluorescent reticulocytes. The immature reticulocyte fraction is a ratio of the quantity of immature reticulocytes to the quantity of reticulocytes in the same measurement sample.

In addition, fluorescent light signal intensity of reticulocyte particles may characterize nucleic acid content in reticulocytes. The more the nucleic acids are, the stronger the fluorescent light is. Therefore, the nucleic acid content in reticulocytes may be calculated by accumulating fluorescent light signals of reticulocyte particles. The formula for calculating the nucleic acid content is as follows:

$$N_{RET} = \sum_{i=1}^{N} FL_{RET-i}$$

Wherein, $N_{RET}$ is characterizing amount of the nucleic acid content in reticulocytes, $FL_{RET-i}$ is fluorescent light signal intensity of the $i^{th}$reticulocyte. The characterizing amount of the nucleic acid content may be used for monitoring the maturation process of red blood cells. The higher the nucleic acid content is, the more the immature reticulocytes detected in blood are or the more immature the reticulocytes are, the closer they are to the orthochromatic normoblasts. To some extents, the characterizing amount of the nucleic acid content can reflect the severity of blood diseases.

The reticulocyte marker information refers to information indicating that reticulocyte particles have been detected, or the reticulocyte marker information may also refer to information indicating that reticulocytes having a certain maturity degree have been detected. The reticulocyte marker information is a qualitative index, for example, when reticulocyte particles or reticulocytes having a certain maturity degree have been detected, the reticulocyte marker information is set as 1; when no predetermined type of reticulocyte particles has been detected, the reticulocyte marker information is set as 0.

In some embodiments, an alarm may also be given according to the reticulocyte information. For example, an alarm is given according to the reticulocyte marker information, that is, an alarm is given when the reticulocyte marker information is set as 1. Or an alarm is given according to the reticulocyte count information, that is, an alarm is given when the reticulocyte count information exceeds a set threshold value. Or an alarm is given according to the high fluorescent reticulocyte count information or the nucleic acid content in reticulocytes.

In another embodiment, the data processor may also identify large platelets according to a forward scattered light-fluorescent light scattergram, and the flow thereof is shown in Fig. 4b. On the basis of Fig. 4a, the following steps are also included:

Step 109, identifying a blood ghost region 32b on the scattergram. The blood ghost region is generally located in the left lower corner region of the scattergram, and the blood ghost region may be set on the scattergram according to experience using gating method. In this solution, the blood ghost region is preset, and fixed. The blood ghost region may not be preset, for example, the blood ghost region is determined according to white blood cell distribution region, and the while blood cell distribution region may be a preset region, or may be a region determined according to actually detected white blood cell distribution. In this solution, the data processor reads the white blood cell distribution region, and then determines the blood ghost region utilizing the white blood cell distribution region. For example, the center of the white blood cell distribution region is shifted toward the left lower direction by a predetermined distance to obtain the center of the blood ghost region, and then a circle or ellipse is drawn according to the center and the predetermined radius of the blood ghost region, so as to obtain the blood ghost region.

Step 110, obtaining the second region 35 and the first region 36 according to the blood ghost region. According to theory and experience, a specific region of the first branch of the blood ghost region may be set as the second region 35, and a specific region of the second branch may be set as the first region 36. As shown in Fig. 3c, the second region 35 and the first region 36 are located in the blood ghost region.

Step 111, identifying particles in the second region 35 as large platelets.

Step 112, obtaining large platelet information according to the identified large platelets. The large platelet information includes at least one of the followings: volume sizes of the large platelets, volume distribution of the large platelets and count information of the large platelets.

The count information of large platelets includes the quantity of large platelets and/or the ratio of large platelets. The quantity of large platelets may be obtained by counting particles in the first region. The ratio of large platelets is a ratio

of the quantity of large platelets to the total number of platelets in the same test sample.

The volume sizes of large platelets are calculated at least according to the forward scattered light signals of large platelets, for example, by any one of the following methods:

1. If both forward scattered light (FSC) and side scattered light (SSC) information of particles are detected, the volume sizes of large platelets may be calculated by utilizing Mie scattering theory, specifically referring to "Forward Light Scattering Of Red Blood Cells", journal 2, Volume 5, Laser Biology, June 1996.
2. If only forward scattered light information is detected, the volume sizes of large platelets may be calculated utilizing formula (1), wherein k is a constant, Vol is volume size of a large platelet, and FSC is forward scattered light of a particle.

$$Vol = k * FSC \text{ ------ } (1)$$

Or the volume sizes of large platelets may also be calculated by utilizing formula (2), wherein k and b are constants.

$$Vol = k*\exp(b*FSC) \text{ -------- } (2)$$

3. If only forward scattered light information is detected, the volume sizes of large platelets may be calculated by formula (3), wherein $\mu$ and $\sigma$ are constants.

$$Vol = [1/(FSC*\sigma(2\pi)1/2)]\exp(-(\ln FSC-\mu)^2/2\sigma^2) \text{ ----- } (3)$$

According to the volume sizes of large platelets, the volume distribution of large platelets may be obtained, for example, a volume distribution histogram of large platelets is generated, as shown in Fig. 6. In some embodiments, the volume distribution information of large platelets may also be obtained by performing segment counting on forward scattering optical axis.

In order to verify the effect of this solution, results obtained by the embodiments of the present invention are compared with results obtained by using BC-6800 blood cell analyzer, as shown in Figs. 7a-7h. Figs. 7a-7d show a correlation between the large platelet count information obtained according to the embodiments of the present invention and the large platelet count information obtained by using BC-6800 blood cell analyzer, and Figs. 7e-7h show a correlation between reticulocyte count information obtained according to the embodiments of the present invention and the reticulocyte count information obtained by using BC-6800 blood cell analyzer.

Embodiments

Blood samples were detected by using white blood cell detection channel of BC-6800 blood cell analyzer produced by Shenzhen Mindray biomedical electronic Co., Ltd (BC-6800 blood cell analyzer for short herein). Reticulocyte channel of BC-6800 blood cell analyzer adopts flow cytometry and can detect reticulocyte count value through forward scattered light and fluorescent light. The scattergram is shown in Fig. 1.

In addition, it can be seen from Fig. 1 that platelets are identified by using forward scattered light signals and fluorescent light signals, and the total number of platelets is obtained. The volume of each platelet is calculated by using Mie scattering theory (Wei Zhang, Yuan Lu, Shiming Du, etc., Mie Scattering Characteristic Analysis Of Spherical Particles, Optical Technology, November 2010: Volume 36, No.6: 936-939) based on forward scattered light signals and side scattered light signals of platelet particles, thereby obtaining the quantity of platelets having different volumes.

Referring to Fig. 7a-7d, 82 samples were selected for a comparative experiment, wherein through confirmation via artificial microscopy, 15 samples were samples containing red blood cell fragments, 5 samples were samples containing small red blood cells, 5 samples were samples containing large platelets and 57 samples were normal samples. Detections were carried out in white blood cell detection channel of Mindray BC-6800 blood cell analyzer by using the solution of the present invention, so as to obtain forward scattered light signals of particles in the second region 35 and the volume distribution data of large platelets. Specifically, forward scattered light signals FSC may be respectively converted into the volume of each particle in this region through formula (1), thereby obtaining the volume distribution data of large platelets, as shown in Fig. 6. The volume distribution data may be present in a digital form or a graphic form, such as volume histogram. Furthermore, based on the volume distribution data of large platelets, the count information of large platelets with volumes above 10fL, 12fL, 15fL and 20fL was respectively measured. The same blood samples were

detected in reticulocyte channel of Mindray BC-6800 blood cell analyzer, and quantities of platelets having different volumes were calculated by using the above methods. In figures, transverse axis represents the count information of large platelets with volumes above 10fL, 12fL, 15fL respectively measured by BC-6800 blood cell analyzer (which is abbreviated as BC-6800 in the figures), and longitudinal axis represents the count information of large platelets with volumes above 10fL, 12fL, 15fL respectively measured by using the solution of the present invention (which is abbreviated as the present invention). It can be seen from the figures that the squares of the relevant coefficients of the two detection methods are 0.94, 0.945, 0.935 and 0.924 respectively.

Referring to Figs. 7e-7h, 88 samples were selected for a comparative experiment, wherein through detection by BC-6800 blood cell analyzer, 25 samples were samples with RET% < 0.5%, 19 samples were samples with 0.5 ≤ RET% < 3% and 44 samples were samples with RET% > 3%. Detections were carried out respectively using the solution of the present invention and BC-6800 blood cell analyzer. The ratio and count value (RET%/RET#) of reticulocytes, low fluorescent ratio (LFR), middle fluorescent ratio (MFR), high fluorescent ratio (HFR), and immature reticulocyte fraction (IRF, the sum of MFR and HFR) can be detected in reticulocyte measurement channel of BC-6800 blood cell analyzer. Through conversion, the low fluorescent reticulocyte count value $LFR\# = RET\#^{*} LFR$, middle fluorescent reticulocyte count value $MFR\# = RET\#^{*}\, \mathrm{M}\, FR$, high fluorescent reticulocyte count value $HFR\# = RET\#^{*}\, \mathrm{H}\, FR$ and immature reticulocyte count value $IRF\# = RET\#^{*} IRF$ can be obtained. In addition, the nucleic acid content $N_{RET}$ of reticulocytes can be characterized by accumulating fluorescent signal intensities of reticulocytes detected by BC-6800 blood cell analyzer. In the figures, transverse axis represents the statistical information of a sum of RET#, IRF#, HFR# and fluorescent light respectively measured by BC-6800 blood cell analyzer (which is abbreviated as BC-6800), and longitudinal axis represents the statistical information of a sum of RET#, IRF#, HFR# and fluorescent light respectively measured by using the solution of the present invention (which is abbreviated as the invention). It can be seen from the figures that the squares of the relevant coefficients of the two detection methods are 0.847, 0.746, 0.737 and 0.864 respectively. It can be seen from the comparison results that the quantity of particles in a specific region can effectively characterize information of reticulocytes in blood.

In other embodiments, the solution of the present invention may also be carried out in nucleated red blood cell detection channel of BC-6800 blood cell analyzer, and similar to white blood cell channel, information of reticulocytes and the quantity of large platelets may also be obtained.

In a further improved embodiment, an alarm may also be given according to the large platelet count information obtained from the above embodiments. For example, when the quantity of large platelets exceeds a set threshold, an alarm prompt may be given by means of sound, or may be given on a display screen by means of words or highlighting display, or may be given on a printed report sheet by means of words or highlighting display.

It should be understood by those skilled in the art that in another embodiment, only two data, namely forward scattered light signal and fluorescent light signal may be collected, that is, each cell particle is characterized by two data. The first region, the second region, the third region, the fourth region and the fifth region may be preset according to experience, or determined according to a determined region. For example, after the blood ghost region is determined, a specific region distanced from the center of the blood ghost region by a first distance and orientation may be determined as the first region for differentiating large platelets, a specific region distanced from the center of the blood ghost region by a second distance and orientation may be determined as the second region for differentiating reticulocytes, a region which is located in the upper one third of the second region is determined as the third region for differentiating high fluorescent reticulocytes, a region which is located in the middle one third of the second region is determined as the fourth region for differentiating middle fluorescent reticulocytes, and an region which is located in the lower one third of the second region is determined as the fifth region for differentiating low fluorescent reticulocytes. In some specific embodiments, the scattergrams may also be displayed on the display screen, and each region may be determined by a user through manual gating according to experience. In these embodiments, the blood ghost region may also not be determined, that is, step 109 is not executed, but the first region, the second region, the third region, the fourth region and the fifth region may be directly specified on the scattergrams.

In some embodiments, a three-dimensional scattergram may also be generated based on forward scattered light information, side scattered light information and fluorescent light information. Then, reticulocyte particles and/or large platelets are identified according to the distribution of particles in the scattergram in forward scattered light dimension and fluorescent light dimension.

These embodiments mainly describe differentiation of reticulocytes and platelets through gating on the scattergrams. In some embodiments, which particles are reticulocytes and which particles are large platelets are identified and judged by comparing with a threshold, for example, a first threshold range and a second threshold range are set for forward scattered light, and a third threshold range and a fourth threshold range are set for fluorescent light. Particles whose forward scattered light falls in the first threshold range and whose fluorescent light falls in the third threshold range are identified as large platelets, particles whose forward scattered light falls in the second threshold range and whose fluorescent light falls in the fourth threshold range are identified as reticulocytes. In these embodiments, it is not needed to generate any scattergram.

In addition, in some embodiments, identification and analysis may be performed only for large platelets, or only for reticulocytes.

Upon deep studies, the applicant has found that after hemolytic treatment of red blood cells in a blood sample, reticulocytes can form reticulocyte particles, which can at least partly be differentiated from the red blood cell fragments on the forward scattered light-fluorescent light scattergram, and the reticulocyte particle information including the quantity of reticulocyte particles can thus be obtained, which is related to the information of reticulocytes in the sample.

Because white blood cell measurement channel which is required to lyse red blood cells is adopted in the embodiments of the present invention, in some embodiments, white blood cell analysis, including classification and count of white blood cells, may be performed utilizing the same measurement data, for example, four classifications of white blood cells (as shown in Fig. 3a, white blood cells are differentiated into four populations, which are respectively lymphocytes, monocytes, neutrophils and eosinophils), classification of basophils, classification of nucleated red blood cells and the like are performed. A scattergram may be generated according to any two of forward scattered light signal, side scattered light signal and fluorescent light signal, and then white blood cells are classified and counted by gating on the scattergram. Those skilled in the art can understand that other measurement channels required to lyse red blood cells during detection, such as nucleated red blood cell channel, may be applied to the method of the present invention, and nucleated red blood cells are identified while identifying reticulocyte particles (shown in Fig. 3d).

In another embodiment, reticulocytes may also be detected by using a hemolytic agent which has a stronger hemolytic ability on red blood cells in combination with a fluorescent dye. The hemolytic agent may be at least one of alkyl glycoside, triterpenoid saponin and steroidal saponin. This kind of hemolytic agents can better reduce the interference of fragments obtained after red blood cells are lysed on large platelets and reticulocyte particles.

A specific hemolytic agent may be a glycoside compound having the general formula I:

$$R\text{-}(CH_2)n\text{-}CH_3 \qquad (I)$$

wherein R is selected from the group consisting of monosaccharide, deoxy monosaccharide and polysaccharide, and n is an integer of 5-17.

The above glycoside compound is capable of quickly lysing red blood cells. The glycoside compound is a compound formed by dehydrating the hemiacetal hydroxyl group of saccharide (or polysaccharide) and the hydroxyl group of alkanol. The glycoside compound in the hemolytic agent of the present invention may be a single compound or a mixture of two or more glycoside compounds in accordance with the above-mentioned general formula.

In the general formula (I), the monosaccharide is not particularly limited. The commonly used monosaccharide may be selected from pentose, methyl pentose and hexose, but is not limited thereto. The pentose comprises such as arabinose, xylose, ribose, lyxose, etc. The methyl pentose comprises such as fusantose, rhamnose, quinovose, etc. The hexose comprises such as glucose, mannose, fructose, galactose and sorbose. The deoxy monosaccharide is also not particularly limited, and comprises such as deoxyribose, deoxyglucose, etc., but is not limited thereto. The polysaccharide comprises such as maltose, sucrose, etc., but is not limited thereto. n is preferably an integer of 6 to 14, more preferably an integer of 7 to 11.

The glycoside compound having the general formula I may specifically be octyl glucoside, nonyl glucoside, decyl glucoside, dodecyl maltoside, tetradecyl maltoside, dodecyl glucoside, preferably octyl glucoside, nonyl glucoside, decyl glucoside and dodecyl maltoside, more preferably decyl glucoside and dodecyl maltoside.

The concentration of the glycoside compound having the general formula I in the hemolytic agent of this embodiment varies according to the properties of the selected glycoside, the reaction time, the reaction temperature and the dosage of other components. Generally, the dosage is within the range from 0.025 g/L to 10 g/L, preferably within the range from 0.1 g/L to 5.0 g/L.

The hemolytic agent in a first embodiment preferably further comprises

a non-ionic surfactant having the general formula II:

$$R_1\text{-}R_2\text{-}(CH_2CH_2O)_m\text{-}H \qquad (II)$$

wherein $R_1$ is C8-C23 alkyl group, $R_2$ is -O-,

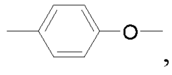,

or -COO-, and m is an integer of 10-50; and

optionally, at least one organic acid or a salt thereof, wherein the organic acid or the salt thereof is selected from the

group consisting of organic acids having at least one carboxyl group or sulfonic acid group and alkali metal salts thereof.

The non-ionic surfactant having the general formula II is capable of binding to cell membranes of white blood cells to a certain extent, so as to achieve an effect of protecting the cell membranes of white blood cells and platelets from being influenced by the aforementioned glycoside compounds, thereby maintaining or substantially maintaining their cell morphologies.

According to a preferred embodiment, in the non-ionic surfactant having the general formula II, $R_1$ is a linear C8-C18 alkyl group. The linear C8-C18 alky group may specifically be octyl, decyl, lauryl, tetradecyl, hexadecyl or stearyl. More preferably, $R_1$ is a linear C12-C16 alkyl group, which may specifically be lauryl, tetradecyl or hexadecyl. $R_2$ is preferably -O-. m is 10~50, preferably 15~30.

Specific examples of the non-ionic surfactants having the general formula II may be cetanol polyoxyethylene (15) ether, dodecanol polyoxyethylene (21) ether, cetanol polyoxyethylene (23) ether, cetanol polyoxyethylene (25) ether and cetanol polyoxyethylene (30) ether, but are not limited thereto.

The concentration of the non-ionic surfactant having the general formula II is not particularly limited, but may be 0.03~1.5 g/L, preferably 0.05~1.0 g/L.

In this embodiment, the non-ionic surfactant may be used as a single substance or a mixture of two or more substances. Depending on the type of used non-ionic surfactant, its concentration in the hemolytic agent also varies. Generally speaking, the concentration of the non-ionic surfactant with a longer alkyl chain and more repeat units in the polyoxyethylene part is relatively low.

In this embodiment, the compounds having the general formula I and the general formula II are used cooperatively, so that on the one hand, the effect of quickly and deeply lysing red blood cells can be achieved and on the other hand, cell membranes of platelets can be protected in order to effectively detect the platelets.

According to the selected compounds having the general formula I and general formula II, their dosage ratio also varies. However, in general, the dosage ratio of the compounds having the general formula I and the general formula II is 1:100 to 1:3, preferably 1:25 to 1:5, and more preferably 1:10 to 1:5.

According to a preferred embodiment, the hemolytic agent may further comprise at least one organic acid or salt thereof to improve the differentiation degree of scattered light of white blood cells. The organic acid is preferably selected from the group consisting of C1-6 alkyl mono-, di-, or tri-carboxylic acid which is unsubstituted or substituted with a hydroxy group or an amino group, C1-6 alkyl sulfonic acid which is unsubstituted or substituted with a hydroxy group or an amino group, C6-10 aryl C1-6 alkyl acid, C6-10 aryl bi(C1-6 alkyl acid) and C6-10 aryl sulfonic acid.

Specific examples of the organic acid and its salt may be formic acid, acetic acid, benzoic acid, citric acid (3-hydroxy-1,3,5-pentyl triacid), malic acid (2-hydroxysuccinic acid), benzenedicarboxylic acid, benzenesulfonic acid, α-naphthalenesulfonic acid, taurine, etc. and their alkali metal salts such as sodium salts and potassium salts, but are not limited thereto.

The concentration of the organic acid or organic acid salt in the hemolytic agent is 0.05g/L~2g/L, preferably 0.1g/L~0.5g/L.

The hemolytic agent of this embodiment may further comprise conventional additives. These additives may be selectively added as required, for example (but not limited to) a buffer agent, a metal chelating agent, an osmotic pressure regulator and a preservative. These reagents are all commonly used reagents in the art, as long as they do not prevent the above components in the hemolytic agent of the present invention from functioning. The buffer agent may be, for example, one selected from phosphoric acid and its salts, citric acid and its salts, TRIS, etc., and is generally a buffer system composed of two or more of them. The metal chelating agent is used as an anticoagulant, for example, commonly used sodium EDTA. The osmotic pressure regulator is usually an inorganic salt such as sodium chloride, sodium sulfate, potassium sulfate, sodium borate, etc. The preservative is for example, isothiazolinone, sodium azide, and imidazolidinyl urea.

The mixing ratio of the hemolytic agent and the blood sample is not particularly limited. For example, the volume mixing ratio of the blood sample to the hemolytic agent may be 1:40~1:60. Hemolytic reaction is carried out for 15~100s, preferably 40-80s, at a temperature such as 40-60°C. The reaction temperature and the reaction time may be adjusted according to specific conditions.

The components of a specific reagent are as follows:

| | |
|---|---|
| Fluorescent dye (structural formula is as follows) | 1.0 ppm |
| Dodecyl maltoside | 0.6 g / L |
| TRIS | 40 Mm |
| Sodium citrate | 5 g/L |
| Polyoxyethylene (23) cetyl ether | 0.5g/l |

(continued)

| | |
|---|---|
| PH | 7.5 |

The structural formula of fluorescent dye:

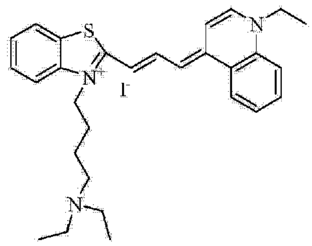

The contribution of the present invention to the prior art lies in that, a solution of detecting reticulocytes and/or large platelets by using a measurement channel required to lyse red blood cells such as white blood cell measurement channel, has been found. The solution can be used to detect the quantity or other information of large platelets, and can also be used to detect the quantity or other information of reticulocytes, for example, more finely classify and count reticulocytes, so that classification and count of white blood cells, reticulocyte information and large platelet information can be obtained according to scattered light information and fluorescent light information collected by one detection, and therefore the technical solution of the present invention has the following technical effects:

Firstly, a measurement channel is saved, which is conducive to the miniaturization of blood analyzers.

Secondly, the detection cost is reduced. On the one hand, white blood cells, reticulocyte particles and large platelets can be identified according to scattered light information and fluorescent light information collected by one detection, which saves the dosage of detection reagents. On the other hand, the price of a reagent for detecting classification and count of white blood cells is much lower than that of a reagent for fine detection of reticulocyte information. First, by screening out suspicious samples among mass samples using the solution of the present, and then finely detecting the suspicious samples as required, the detection cost of mass samples is thereby reduced.

Thirdly, the detection time is saved. In one detection channel, white blood cell information, reticulocyte information and platelet information are provided.

It can be understood by those skilled in the art that all or part of the steps of various methods in the above embodiments may be completed by instructing related hardware via programs, these programs may be stored in a computer-readable storage medium which may include a read-only memory, a random memory, a disk or an optical disk. For example, a program is stored in a memory of an analyzer, when it is needed to detect a sample, the program in the memory is executed through a processor so as to realize the above steps. Particularly, in the practical implementation process of the present invention, steps 107-114 and 206-211 in the above embodiments may be written into independent programs which may be stored on a server, disk, optical disk and flash disk, and may be saved in the memory of the local analyzer by downloading, or may update the version of the system of the local analyzer by downloading. When it is needed to detect a sample, the program in the memory is executed through a processor, so as to achieve the functions of steps 107-114 and steps 206-211.

## Claims

**1.** A blood analyzer, comprising:

a test sample preparation apparatus for preparing a test sample for measurement, wherein the test sample preparation apparatus at least comprises a reaction cell (21), and the reaction cell (21) is configured for providing a place for mixing and incubation of a test blood sample, a fluorescence staining agent and a hemolytic agent, and the mixing and incubation causes red blood cells in the test blood sample to be lysed by the hemolytic agent and causes cell particles in the sample to be stained by the fluorescence staining agent, so as to prepare the test sample for measurement;

a flow chamber (22c) configured for providing an area for the cell particles in the test sample to pass through one by one and to be irradiated with light;

a measurement apparatus comprising a light source (22a) and an optical detection device (22f, 22k, 22g), wherein the light source (22a) is configured for emitting a light beam to irradiate the flow chamber (22c), the optical detection device (22f, 22k, 22g) is configured for receiving scattered light and fluorescent light generated by the cell particles in the test sample under the irradiation of light and outputting scattered light information and

fluorescent light information, and the scattered light information at least comprises first angle scattered light information which is used for reflecting volume size information of the cell particles; and
**characterized by**
a data processor (25) configured for identifying reticulocyte particles in the test sample treated by the fluorescence staining agent and the hemolytic agent according to the first angle scattered light information and the fluorescent light information of the cell particles in the test sample;
wherein the data processor (25) is further configured for classifying and/or counting white blood cells in the test sample according to the scattered light information and the fluorescent light information of the cell particles in the test sample.

**2.** The blood analyzer according to claim 1, wherein the data processor (25) is configured for generating a scatter diagram at least according to the first angle scattered light information and the fluorescent light information of the cell particles, and identifying particles in a first region (36) of the scatter diagram as reticulocyte particles, wherein the first region refers to a region in a blood ghost region (32b) where fluorescent light signal intensities are relatively large and first angle scattered light signal intensities are relatively small.

**3.** The blood analyzer according to claim 1, wherein the data processor (25) is further configured for acquiring reticulocyte information according to the identified reticulocyte particles.

**4.** The blood analyzer according to claim 3, wherein the reticulocyte information comprises at least one of the followings: reticulocyte marker information, reticulocyte count information, high fluorescent reticulocyte count information, middle fluorescent reticulocyte count information, low fluorescent reticulocyte count information, immature reticulocyte count information, and nucleic acid content in reticulocytes.

**5.** The blood analyzer according to any one of claims 3-4, wherein the data processor (25) is further configured for giving an alarm at least according to the reticulocyte information.

**6.** The blood analyzer according to any one of claims 1-5, wherein the data processor (25) is further configured for identifying large platelets according to the first angle scattered light information and the fluorescent light information.

**7.** The blood analyzer according to claim 6, wherein the data processor (25) is configured for generating a scatter diagram according to the first angle scattered light information and the fluorescent light information, and identifying particles in a second region (35) of the scatter diagram as large platelets, wherein the second region (35) refers to a region in a blood ghost region (32b) where fluorescent light signal intensities are relatively small and first angle scattered light signal intensities are relatively large.

**8.** A blood analysis method, comprising:

staining (102) a test blood sample with a fluorescence staining agent, and performing hemolytic reaction (101) on the test blood sample with a hemolytic agent to lyse red blood cells in the test blood sample, thereby preparing a test sample for measurement;
making (104) cell particles in the test sample pass through a flow chamber one by one and be irradiated with light;
receiving (105) scattered light and fluorescent light generated by the cell particles in the test sample under the irradiation of light and outputting scattered light information and fluorescent light information, wherein the scattered light information at least comprises first angle scattered light information which is used for reflecting volume size information of the cell particles; and **characterized by**
identifying (113, 114) reticulocyte particles in the test sample treated by the fluorescence staining agent and the hemolytic agent according to the first angle scattered light information and the fluorescent light information of the cell particles in the test sample;
wherein the method further comprises: classifying and/or counting white blood cells in the test sample according to the scattered light information and the fluorescent light information of the cell particles in the test sample.

**9.** The method according to claim 8, wherein identifying (113, 114) reticulocyte particles according to the first angle scattered light information and the fluorescent light information of the cell particles comprises: generating a scatter diagram according to the first angle scattered light information and the fluorescent light information of the cell particles, identifying (113) particles in a first region of the scatter diagram as reticulocyte particles, wherein the first region refers to a region in a blood ghost region (32b) where fluorescent light signal intensities are relatively large and first angle scattered light signal intensities are relatively small.

**10.** The method according to claim 8 or 9, further comprising: acquiring reticulocyte information according to the identified reticulocyte particles.

**11.** The method according to claim 10, wherein the reticulocyte information comprises at least one of the followings: reticulocyte marker information, reticulocyte count information, high fluorescent reticulocyte count information, middle fluorescent reticulocyte count information, low fluorescent reticulocyte count information, immature reticulocyte count information and nucleic acid content in reticulocytes.

**12.** The method according to claim 10 or 11, further comprising: giving an alarm at least according to the reticulocyte information.

**13.** The method according to any one of claims 8-12, further comprising: identifying large platelets according to the first angle scattered light information and the fluorescent light information of the cell particles.

**14.** The method according to claim 13, wherein identifying large platelets according to the first angle scattered light information and the fluorescent light information of the cell particles comprises: generating a scatter diagram according to the first angle scattered light information and the fluorescent light information of the cell particles, identifying particles in a second region of the scatter diagram as large platelets, wherein the second region refers to a region in a blood ghost region (32b) where fluorescent light signal intensities are relatively small and first angle scattered light signal intensities are relatively large.

**Patentansprüche**

**1.** Ein Blutanalysegerät, das Folgendes umfasst:

eine Vorrichtung zur Probenvorbereitung zur Vorbereitung einer Probe für eine Messung, wobei die Vorrichtung zur Probenvorbereitung mindestens eine Reaktionszelle (21) umfasst und die Reaktionszelle (21) so ausgelegt ist, dass sie einen Ort zum Mischen und Inkubieren einer Blutprobe, eines Fluoreszenzfärbemittels und eines Hämolytikums bietet. Durch das Mischen und Inkubieren werden die roten Blutkörperchen in der Blutprobe mittels des Hämolytikums lysiert und die Zellpartikel in der Probe durch das Fluoreszenzfärbemittel angefärbt, um die Probe für die Messung vorzubereiten;
eine Durchflusskammer (22c), die dazu ausgelegt ist, dass sie einen Bereich bereitstellt, den die Zellpartikel in der Probe einzeln durchlaufen und dort mit Licht bestrahlt werden;
eine Messvorrichtung mit einer Lichtquelle (22a) und einer optischen Detektionsvorrichtung (22f, 22k, 22g), wobei die Lichtquelle (22a) dazu ausgelegt ist, einen Lichtstrahl zur Bestrahlung der Durchflusskammer (22c) auszusenden, die optische Detektionsvorrichtung (22f, 22k, 22g) Streulicht und Fluoreszenzlicht empfängt, das von den Zellpartikeln in der Testprobe erzeugt wird, und die Streulichtinformationen mindestens Informationen zum ersten Streulichtwinkel umfassen, die zur Wiedergabe von Volumengrößeninformationen der Zellpartikel verwendet werden;
**dadurch gekennzeichnet, dass**
ein Datenprozessor (25) dazu ausgelegt ist, Retikulozytenpartikel in der mit dem Fluoreszenzfärbemittel und dem Hämolytikum behandelten Testprobe anhand der Informationen zum ersten Streulichtwinkel und der Fluoreszenzlichtinformationen der Zellpartikel in der Testprobe zu identifizieren;
wobei der Datenprozessor (25) ferner dazu ausgelegt ist, weiße Blutkörperchen in der Testprobe anhand der Streulichtinformationen und der Fluoreszenzlichtinformationen der Zellpartikel in der Testprobe zu klassifizieren und/oder zu zählen.

**2.** Das Blutanalysegerät gemäß Anspruch 1, wobei der Datenprozessor (25) dazu konfiguriert ist, ein Streudiagramm zumindest entsprechend der Informationen zum ersten Streulichtwinkel und der Fluoreszenzlichtinformation der Zellpartikel zu erzeugen und Partikel in einem ersten Bereich (36) des Streudiagramms als Retikulozytenpartikel zu identifizieren, wobei sich der erste Bereich auf einen Bereich in einer Blutgeisterregion (32b) bezieht, in dem die Fluoreszenzlichtsignalintensitäten relativ groß und die Signalintensitäten des ersten Streulichtwinkels relativ klein sind.

**3.** Das Blutanalysegerät gemäß Anspruch 1, wobei der Datenprozessor (25) ferner dazu ausgelegt ist, Retikulozyteninformationen entsprechend den identifizierten Retikulozytenpartikeln zu erfassen.

4. Das Blutanalysegerät gemäß Anspruch 3, wobei die Retikulozyteninformationen mindestens eines der folgenden Elemente umfassen: Retikulozytenmarkerinformationen, Retikulozytenzahlinformationen, Informationen zur Zahl hochfluoreszierender Retikulozyten, Informationen zur Zahl mittelfluoreszierender Retikulozyten, Informationen zur Zahl niedrigfluoreszierender Retikulozyten, Informationen zur Zahl unreifer Retikulozyten und Nukleinsäuregehalt in Retikulozyten.

5. Das Blutanalysegerät gemäß einem der Ansprüche 3-4, wobei der Datenprozessor (25) ferner dazu ausgelegt ist, zumindest entsprechend den Retikulozyteninformationen einen Alarm auszulösen.

6. Das Blutanalysegerät gemäß einem der Ansprüche 1-5, wobei der Datenprozessor (25) ferner dazu ausgelegt ist, große Blutplättchen entsprechend den Informationen zum ersten Streulichtwinkel und den Fluoreszenzlichtinformationen zu identifizieren.

7. Das Blutanalysegerät gemäß Anspruch 6, wobei der Datenprozessor (25) dazu ausgelegt ist, dass er ein Streudiagramm anhand der Informationen zum ersten Streulichtwinkel und zum Fluoreszenzlicht generiert und Partikel in einem zweiten Bereich (35) des Streudiagramms als große Blutplättchen identifiziert, wobei sich der zweite Bereich (35) auf einen Bereich in einem Blutgeisterbereich (32b) bezieht, in dem die Fluoreszenzlichtsignalintensitäten relativ gering und die Signalintensitäten des ersten Streulichtwinkels relativ groß sind.

8. das Färben (102) einer Testblutprobe mit einem Fluoreszenzfärbemittel und das Durchführen einer hämolytischen Reaktion (101) an der Testblutprobe mit einem hämolytischen Mittel, um rote Blutkörperchen in der Testblutprobe zu lysieren und so eine Testprobe für die Messung vorzubereiten;

   das Veranlassen (104) von Zellpartikeln in der Testprobe, einzeln durch eine Durchflusskammer zu strömen und dort mit Licht bestrahlt zu werden;
   das Empfangen (105) von Streulicht und Fluoreszenzlicht, das von den Zellpartikeln in der Testprobe unter Lichteinstrahlung erzeugt wird, und das Ausgeben von Streulichtinformationen und Fluoreszenzlichtinformationen, wobei die Streulichtinformationen zumindest Informationen zum ersten Streulichtwinkel umfassen, die zum Reflektieren von Volumengrößeninformationen der Zellpartikel verwendet werden; **gekennzeichnet durch**
   das Identifizieren (113, 114) von Retikulozytenpartikeln in der mit dem Fluoreszenzfärbemittel und dem Hämolytikum behandelten Testprobe anhand der Informationen zum ersten Streulichtwinkel und der Fluoreszenzlichtinformationen der Zellpartikel in der Testprobe;
   wobei das Verfahren ferner Folgendes umfasst: das Klassifizieren und/oder Zählen von weißen Blutkörperchen in der Testprobe anhand der Streulichtinformationen und der Fluoreszenzlichtinformationen der Zellpartikel in der Testprobe.

9. Das Verfahren gemäß Anspruch 8, wobei das Identifizieren (113, 114) von Retikulozytenpartikeln anhand der Informationen zum ersten Streulichtwinkel und der Fluoreszenzlichtinformationen der Zellpartikel Folgendes umfasst: das Generieren eines Streudiagramms anhand der Informationen zum ersten Streulichtwinkel und der Fluoreszenzlichtinformationen der Zellpartikel, das Identifizieren (113) von Partikeln in einem ersten Bereich des Streudiagramms als Retikulozytenpartikel, wobei sich der erste Bereich auf einen Bereich in einem Blutgeisterbereich (32b) bezieht, in dem die Fluoreszenzlichtsignalintensitäten relativ groß und die Signalintensitäten des ersten Streulichtwinkels relativ klein sind.

10. Das Verfahren gemäß Anspruch 8 oder 9, das weiterhin Folgendes umfasst: das Erfassen von Retikulozyteninformationen anhand der identifizierten Retikulozytenpartikel.

11. Das Verfahren gemäß Anspruch 10, wobei die Retikulozyteninformationen mindestens eines der folgenden Elemente umfassen: Retikulozytenmarkerinformationen, Retikulozytenzahlinformationen, Informationen zur Zahl hochfluoreszierender Retikulozyten, Informationen zur Zahl mittelfluoreszierender Retikulozyten, Informationen zur Zahl niedrigfluoreszierender Retikulozyten, Informationen zur Zahl unreifer Retikulozyten und Nukleinsäuregehalt in Retikulozyten.

12. Das Verfahren gemäß Anspruch 10 oder 11, das weiterhin Folgendes umfasst: das Auslösen eines Alarms zumindest anhand der Retikulozyteninformationen.

13. Das Verfahren gemäß einem der Ansprüche 8-12, das weiterhin Folgendes umfasst: das Identifizieren großer Blutplättchen anhand der Informationen zum ersten Streulichtwinkel und der Fluoreszenzlichtinformationen der

Zellpartikel.

14. Das Verfahren gemäß Anspruch 13, wobei das Identifizieren großer Blutplättchen entsprechend den Informationen zum ersten Streulichtwinkel und den Fluoreszenzlichtinformationen der Zellpartikel Folgendes umfasst: das Generieren eines Streudiagramms entsprechend den Informationen zum ersten Streulichtwinkel und den Fluoreszenzlichtinformationen der Zellpartikel, das Identifizieren von Partikeln in einem zweiten Bereich des Streudiagramms als große Blutplättchen, wobei sich der zweite Bereich auf einen Bereich in einem Blutgeisterbereich (32b) bezieht, in dem die Fluoreszenzlichtsignalintensitäten relativ gering und die Signalintensitäten des ersten Streulichtwinkels relativ groß sind..

## Revendications

1. Analyseur de sang, comprenant :

   un dispositif de préparation d'échantillon d'essai destiné à préparer un échantillon d'essai pour mesure, où le dispositif de préparation d'échantillon d'essai comprend au moins une cellule de réaction (21), et la cellule de réaction (21) est configurée pour fournir un endroit pour le mélange et l'incubation d'un échantillon de sang d'essai, d'un agent de coloration de fluorescence et d'un agent hémolytique, et le mélange et l'incubation amènent des globules rouges dans l'échantillon de sang d'essai à être lysés par l'agent hémolytique et amènent des particules cellulaires dans l'échantillon à être colorées par l'agent de coloration de fluorescence, de sorte à préparer l'échantillon d'essai pour mesure ;
   une chambre d'écoulement (22c) configurée pour fournir une zone permettant aux particules cellulaires dans l'échantillon d'essai de passer une à une et d'être irradiée par de la lumière ;
   un dispositif de mesure comprenant une source de lumière (22a) et un dispositif de détection optique (22f, 22k, 22g), où la source de lumière (22a) est configurée pour émettre un faisceau lumineux pour irradier la chambre d'écoulement (22c), le dispositif de détection optique (22f, 22k, 22g) est configuré pour recevoir la lumière diffusée et la lumière de fluorescence générées par les particules cellulaires dans l'échantillon d'essai sous l'irradiation de lumière et fournir des informations de lumière diffusée et des informations de lumière de fluorescence, et les informations de lumière diffusée comprennent au moins des informations de lumière diffusée à un premier angle lesquelles sont utilisées pour refléter des informations de taille de volume des particules cellulaires ; et
   **caractérisé par**
   un processeur de données (25) configuré pour identifier des particules de réticulocytes dans l'échantillon d'essai traité par l'agent de coloration de fluorescence et l'agent hémolytique selon les informations de lumière diffusée à un premier angle et les informations de lumière de fluorescence des particules cellulaires dans l'échantillon d'essai ;
   où le processeur de données (25) est en outre configuré pour classer et/ou dénombrer des globules blancs dans l'échantillon d'essai selon les informations de lumière diffusée et les informations de lumière de fluorescence des particules cellulaires dans l'échantillon d'essai.

2. Analyseur de sang selon la revendication 1, dans lequel le processeur de données (25) est configuré pour générer un graphique en nuage de points au moins selon les informations de lumière diffusée à un premier angle et les informations de lumière de fluorescence des particules cellulaires, et identifier des particules dans une première région (36) du graphique en nuage de points comme étant des particules de réticulocytes, où la première région se rapporte à une région dans une région fantôme de sang (32b) où les intensités de signal de lumière de fluorescence sont relativement importantes et les intensités de signal de lumière diffusée à un premier angle sont relativement faibles.

3. Analyseur de sang selon la revendication 1, dans lequel le processeur de données (25) est en outre configuré pour acquérir des informations de réticulocytes selon les particules de réticulocytes identifiées.

4. Analyseur de sang selon la revendication 3, dans lequel les informations de réticulocytes comprennent au moins l'une des suivantes : information de marqueur de réticulocytes, information de nombre de réticulocytes, information de nombre de réticulocytes hautement fluorescents, information de nombre de réticulocytes moyennement fluorescents, information de nombre de réticulocytes faiblement fluorescents, information de nombre de réticulocytes immatures, et teneur en acide nucléique dans des réticulocytes.

5. Analyseur de sang selon l'une quelconque des revendications 3 et 4, dans lequel le processeur de données (25) est en outre configuré pour délivrer une alarme au moins selon les informations de réticulocytes.

6. Analyseur de sang selon l'une quelconque des revendications 1 à 5, dans lequel le processeur de données (25) est en outre configuré pour identifier des plaquettes de grande dimension selon les informations de lumière diffusée à un premier angle et les informations de lumière de fluorescence.

7. Analyseur de sang selon la revendication 6, dans lequel le processeur de données (25) est configuré pour générer un graphique en nuage de points selon les informations de lumière diffusée à un premier angle et les informations de lumière de fluorescence, et identifier des particules dans une seconde région (35) du graphique en nuage de points comme étant des plaquettes de grande dimension, où la seconde région (35) se rapporte à une région dans une région fantôme de sang (32b) où les intensités de signal de lumière de fluorescence sont relativement faibles et les intensités de signal de lumière diffusée à un premier angle sont relativement importantes.

8. Procédé d'analyse de sang, comprenant les étapes consistant à :

colorer (102) un échantillon de sang d'essai avec un agent de coloration de fluorescence, et réaliser une réaction hémolytique (101) sur l'échantillon de sang d'essai avec un agent hémolytique pour lyser des globules rouges dans l'échantillon de sang d'essai, en préparant ainsi un échantillon d'essai pour mesure ;
amener (104) des particules cellulaires dans l'échantillon d'essai à traverser une chambre d'écoulement une à une et à être irradiées par de la lumière ;
recevoir (105) de la lumière diffusée et de la lumière de fluorescence générées par les particules cellulaires dans l'échantillon d'essai sous l'irradiation de lumière et fournir des informations de lumière diffusée et des informations de lumière de fluorescence, où les informations de lumière diffusée comprennent au moins des informations de lumière diffusée à un premier angle lesquelles sont utilisées pour refléter des informations de taille de volume des particules cellulaires ; et **caractérisé par**
le fait d'identifier (113, 114) des particules de réticulocytes dans l'échantillon d'essai traité par l'agent de coloration de fluorescence et l'agent hémolytique selon les informations de lumière diffusée à un premier angle et les informations de lumière de fluorescence des particules cellulaires dans l'échantillon d'essai ;
où le procédé comprend en outre : le fait de classer et/ou dénombrer des globules blancs dans l'échantillon d'essai selon les informations de lumière diffusée et les informations de lumière de fluorescence des particules cellulaires dans l'échantillon d'essai.

9. Procédé selon la revendication 8, dans lequel le fait d'identifier (113, 114) des particules de réticulocytes selon les informations de lumière diffusée à un premier angle et les informations de lumière de fluorescence des particules cellulaires comprend : le fait de générer un graphique en nuage de points selon les informations de lumière diffusée à un premier angle et les informations de lumière de fluorescence des particules cellulaires, d'identifier (113) des particules dans une première région du graphique en nuage de points comme étant des particules de réticulocytes, où la première région se rapporte à une région dans une région fantôme de sang (32b) où les intensités de signal de lumière de fluorescence sont relativement importantes et les intensités de signal de lumière diffusée à un premier angle sont relativement faibles.

10. Procédé selon la revendication 8 ou 9, comprenant en outre : le fait d'acquérir des informations de réticulocytes selon les particules de réticulocytes identifiées.

11. Procédé selon la revendication 10, dans lequel les informations de réticulocytes comprennent au moins l'une des suivantes : information de marqueur de réticulocytes, information de nombre de réticulocytes, information de nombre de réticulocytes hautement fluorescents, information de nombre de réticulocytes moyennement fluorescents, information de nombre de réticulocytes faiblement fluorescents, information de nombre de réticulocytes immatures et teneur en acide nucléique dans des réticulocytes.

12. Procédé selon la revendication 10 ou 11, comprenant en outre : le fait de délivrer une alarme au moins selon les informations de réticulocytes.

13. Procédé selon l'une quelconque des revendications 8 à 12, comprenant en outre : le fait d'identifier des plaquettes de grande dimension selon les informations de lumière diffusée à un premier angle et les informations de lumière de fluorescence des particules cellulaires.

**14.** Procédé selon la revendication 13, dans lequel le fait d'identifier des plaquettes de grande dimension selon les informations de lumière diffusée à un premier angle et les informations de lumière de fluorescence des particules cellulaires comprend : le fait de générer un graphique en nuage de points selon les informations de lumière diffusée à un premier angle et les informations de lumière de fluorescence des particules cellulaires, d'identifier des particules dans une seconde région du graphique en nuage de points comme étant des plaquettes de grande dimension, où la seconde région se rapporte à une région dans une région fantôme de sang (32b) où les intensités de signal de lumière de fluorescence sont relativement faibles et les intensités de signal de lumière diffusée à un premier angle sont relativement importantes.

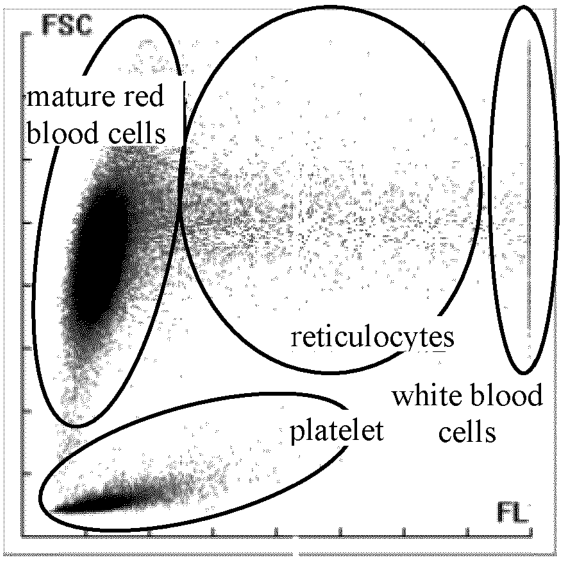
FSC
mature red
blood cells
reticulocytes
white blood
cells
platelet
FL

Fig. 1

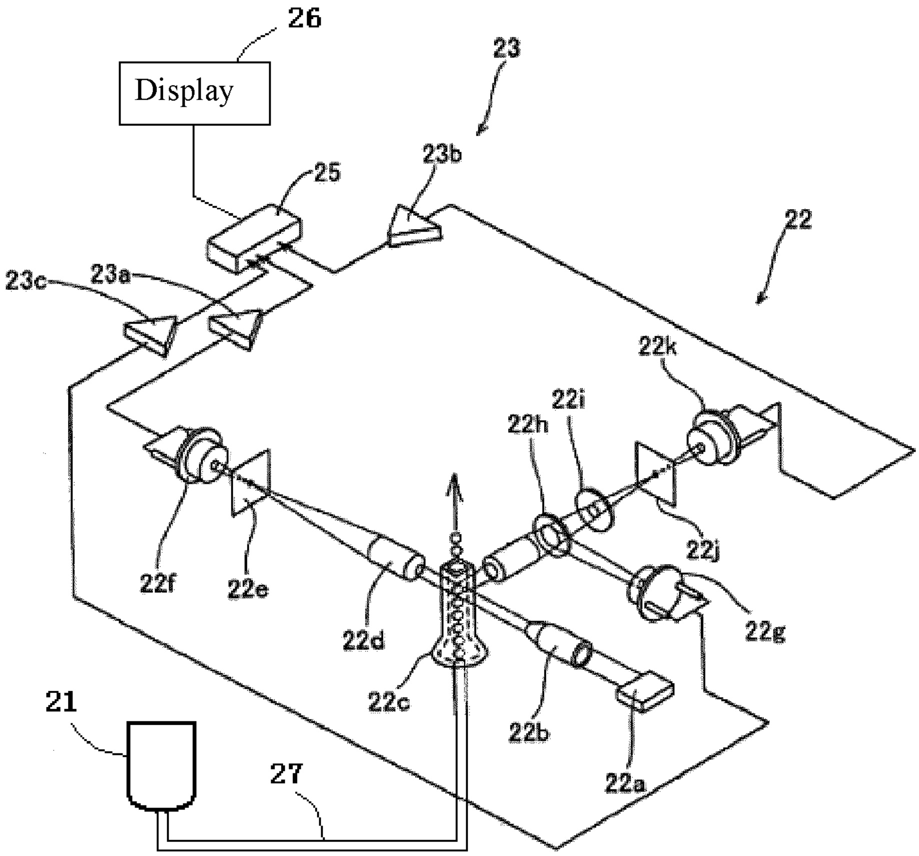
26
Display
23
23b
25
22
23c
23a
22k
22i
22h
22j
22f
22e
22d
22g
21
22c
22b
27
22a

FIG. 2

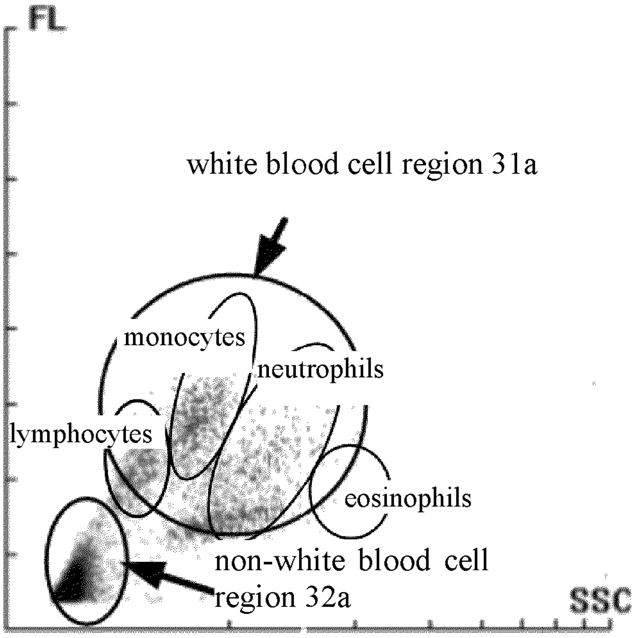
FL
white blood cell region 31a
monocytes
neutrophils
lymphocytes
eosinophils
non-white blood cell region 32a
SSC

FIG. 3a

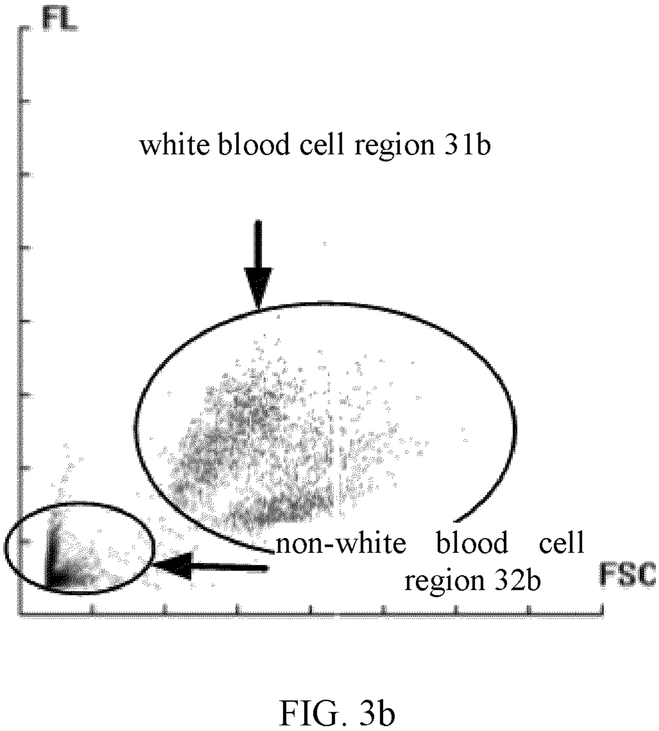
FL
white blood cell region 31b
non-white blood cell region 32b
FSC

FIG. 3b

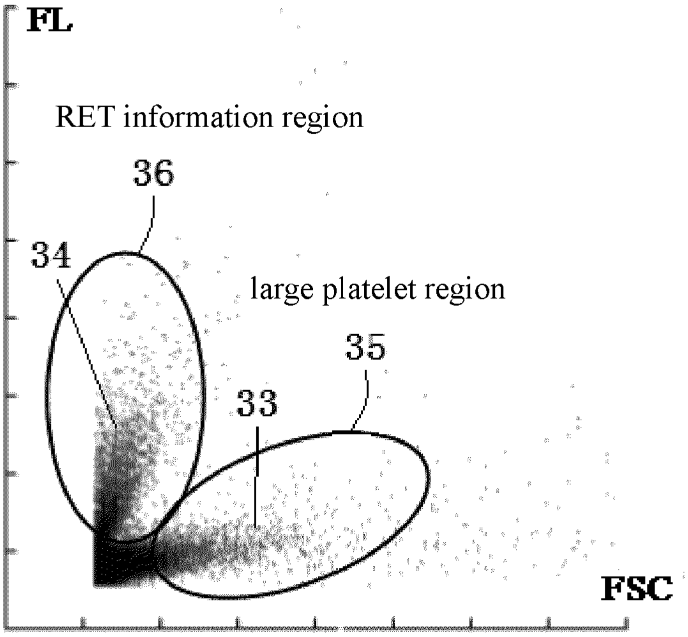
FL
RET information region
36
34
large platelet region
35
33
FSC

FIG. 3c

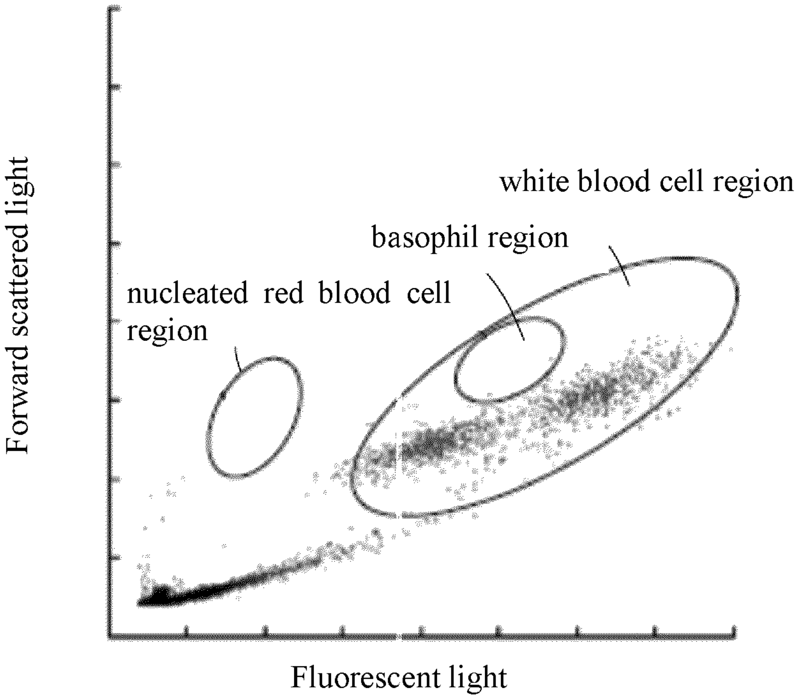
Forward scattered light
white blood cell region
basophil region
nucleated red blood cell region
Fluorescent light

FIG. 3d

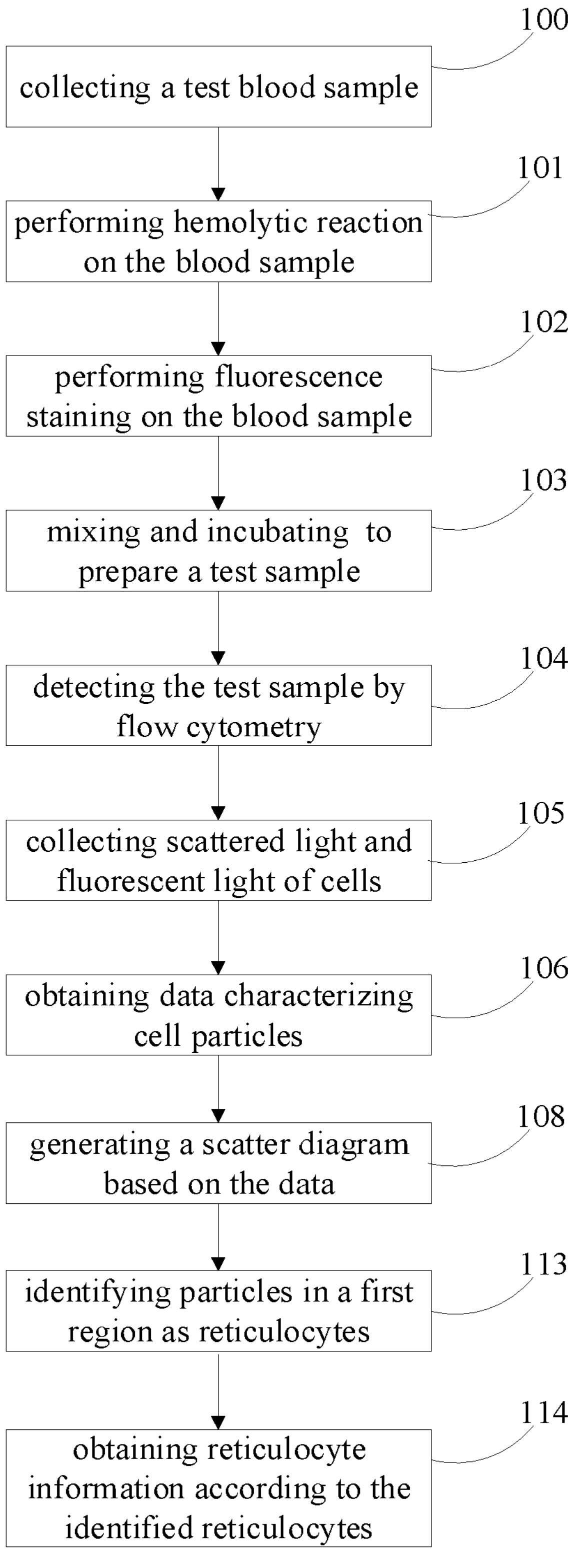
100
collecting a test blood sample
101
performing hemolytic reaction on the blood sample
102
performing fluorescence staining on the blood sample
103
mixing and incubating to prepare a test sample
104
detecting the test sample by flow cytometry
105
collecting scattered light and fluorescent light of cells
106
obtaining data characterizing cell particles
108
generating a scatter diagram based on the data
113
identifying particles in a first region as reticulocytes
114
obtaining reticulocyte information according to the identified reticulocytes

FIG. 4a

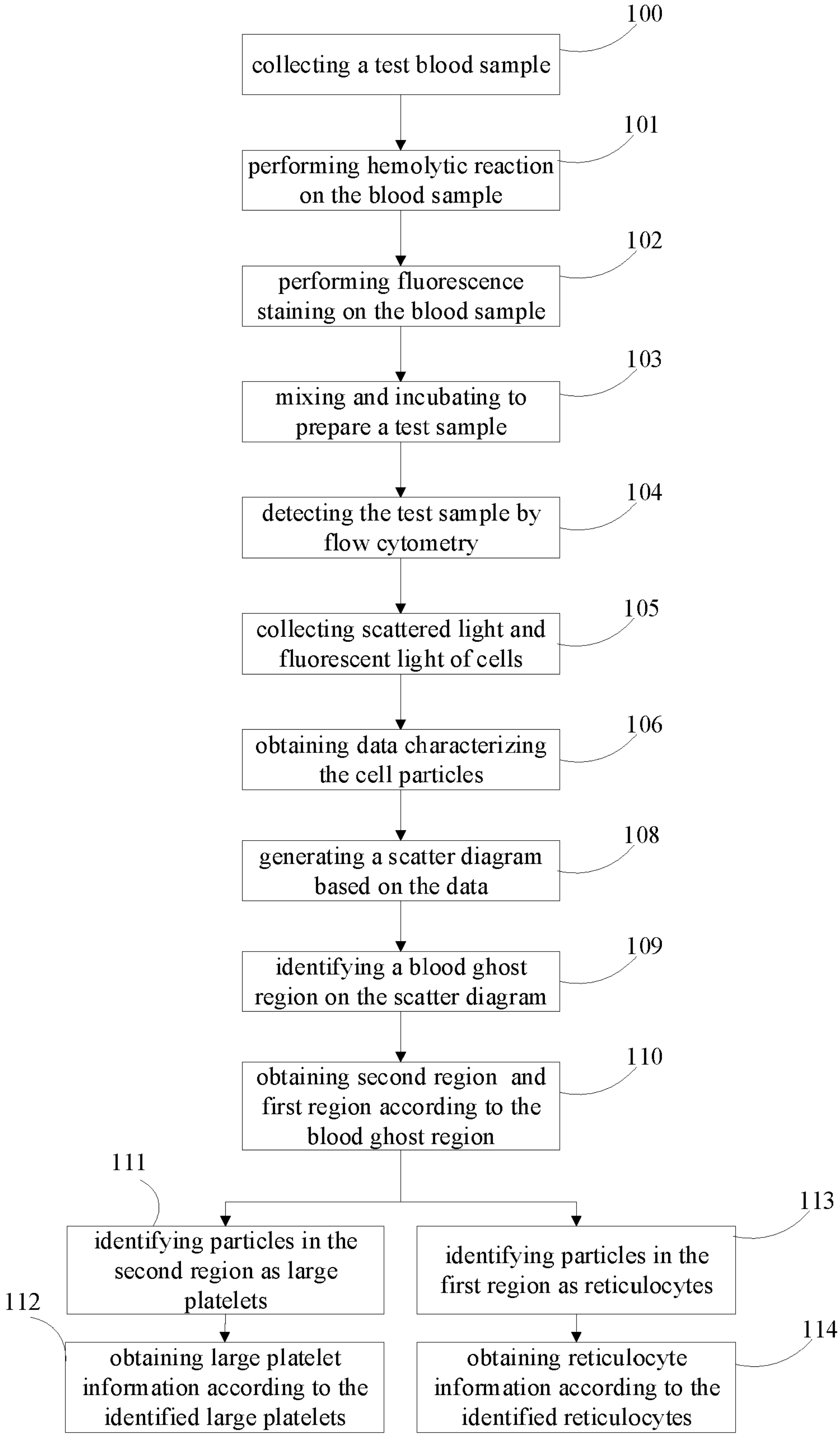
100
collecting a test blood sample
101
performing hemolytic reaction on the blood sample
102
performing fluorescence staining on the blood sample
103
mixing and incubating to prepare a test sample
104
detecting the test sample by flow cytometry
105
collecting scattered light and fluorescent light of cells
106
obtaining data characterizing the cell particles
108
generating a scatter diagram based on the data
109
identifying a blood ghost region on the scatter diagram
110
obtaining second region and first region according to the blood ghost region
111
identifying particles in the second region as large platelets
113
identifying particles in the first region as reticulocytes
112
obtaining large platelet information according to the identified large platelets
114
obtaining reticulocyte information according to the identified reticulocytes

FIG. 4b

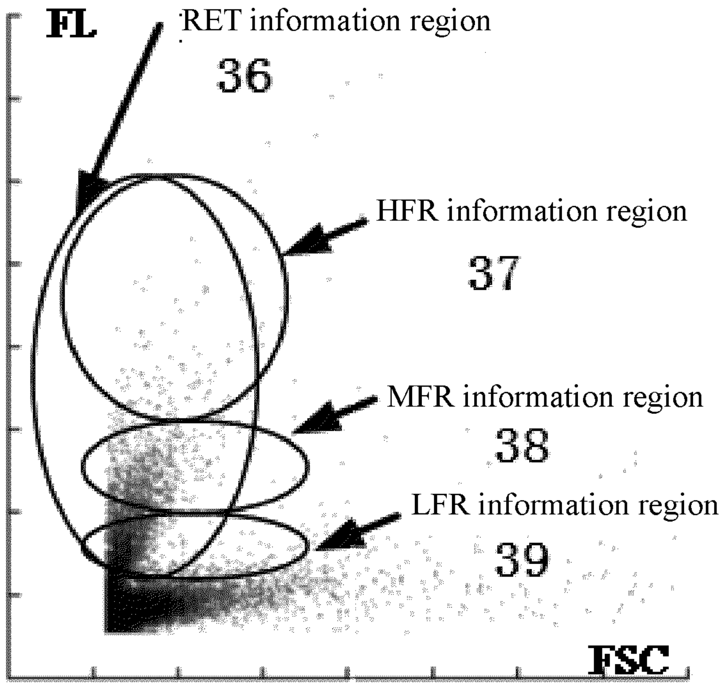
FL
RET information region
36
HFR information region
37
MFR information region
38
LFR information region
39
FSC

FIG. 5

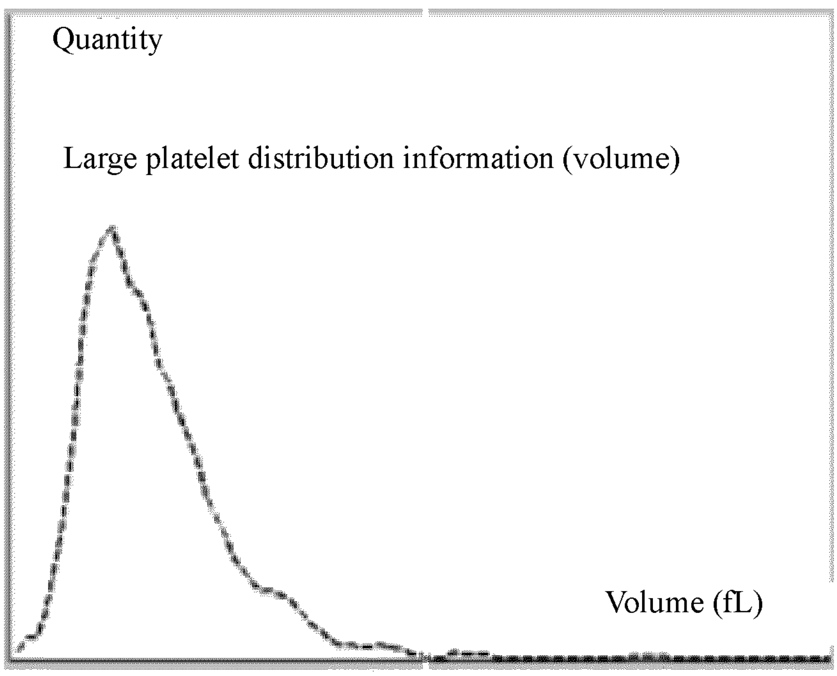
Quantity
Large platelet distribution information (volume)
Volume (fL)

FIG. 6

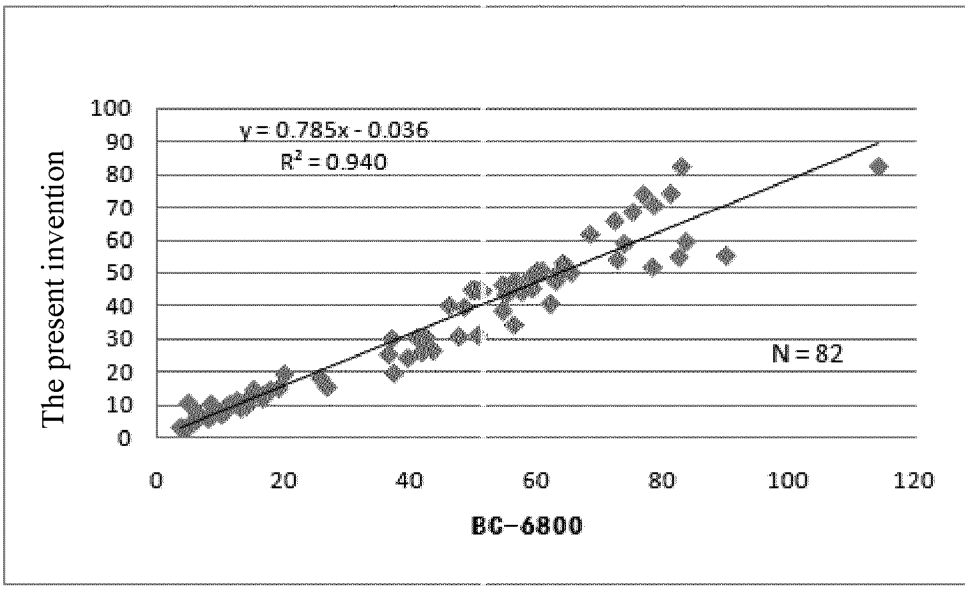
y = 0.785x - 0.036
$R^2$ = 0.940
N = 82
The present invention
BC-6800
100
90
80
70
60
50
40
30
20
10
0
0
20
40
60
80
100
120

FIG. 7a

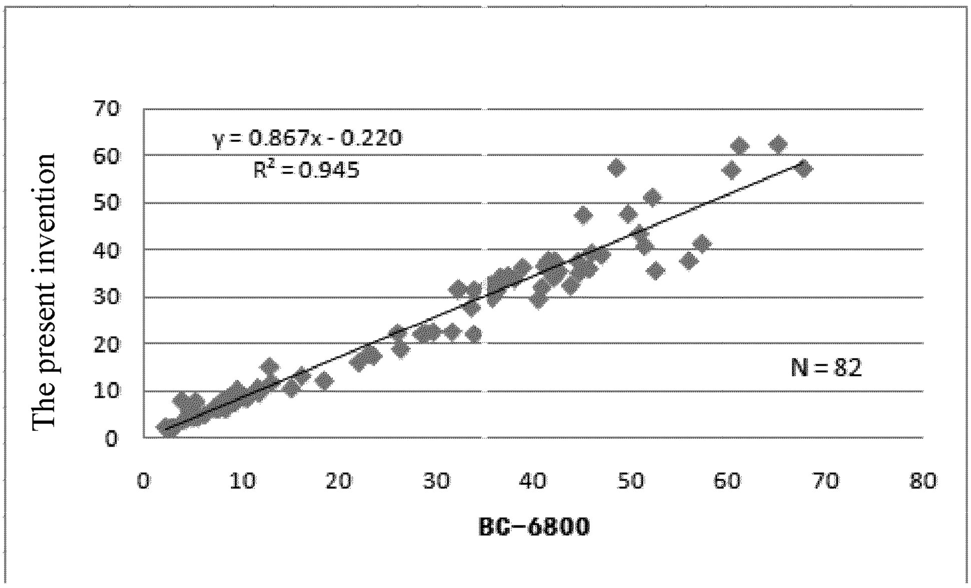
y = 0.867x - 0.220
$R^2$ = 0.945
N = 82
The present invention
BC-6800
70
60
50
40
30
20
10
0
0
10
20
30
40
50
60
70
80

FIG. 7b

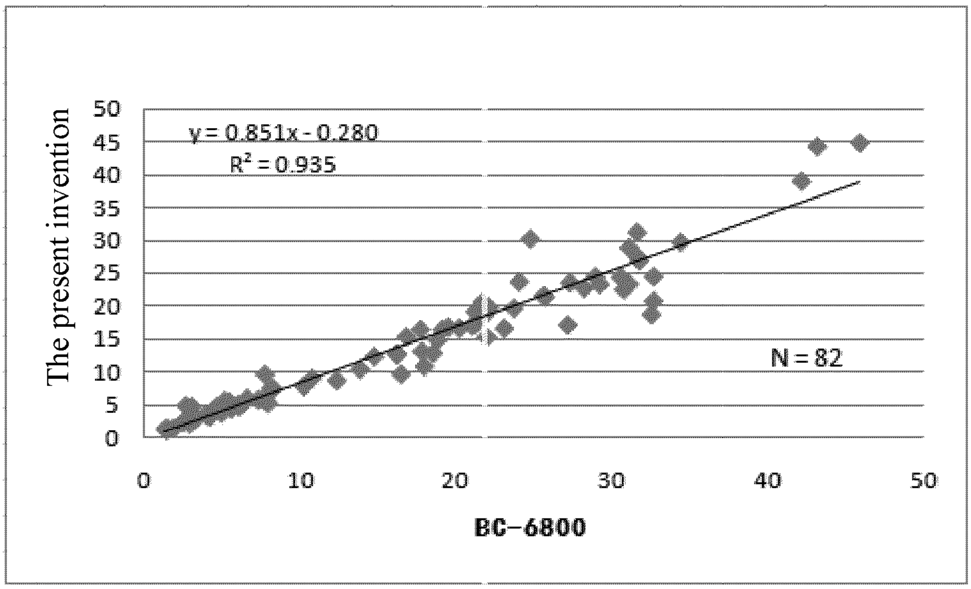

y = 0.851x - 0.280
R² = 0.935
N = 82
The present invention
BC-6800
0
5
10
15
20
25
30
35
40
45
50
0
10
20
30
40
50


FIG. 7c

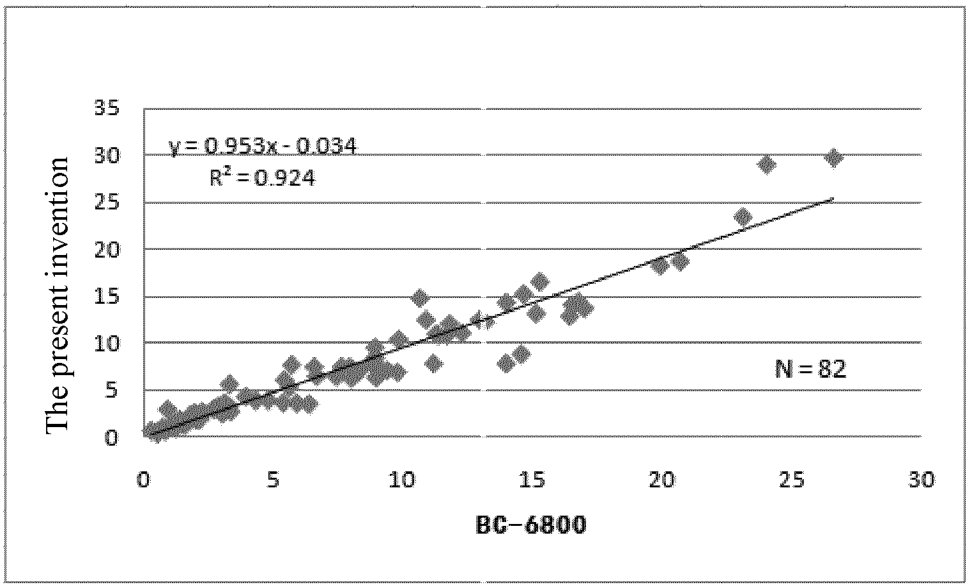

y = 0.953x - 0.034
R² = 0.924
N = 82
The present invention
BC-6800
0
5
10
15
20
25
30
35
0
5
10
15
20
25
30


FIG. 7d

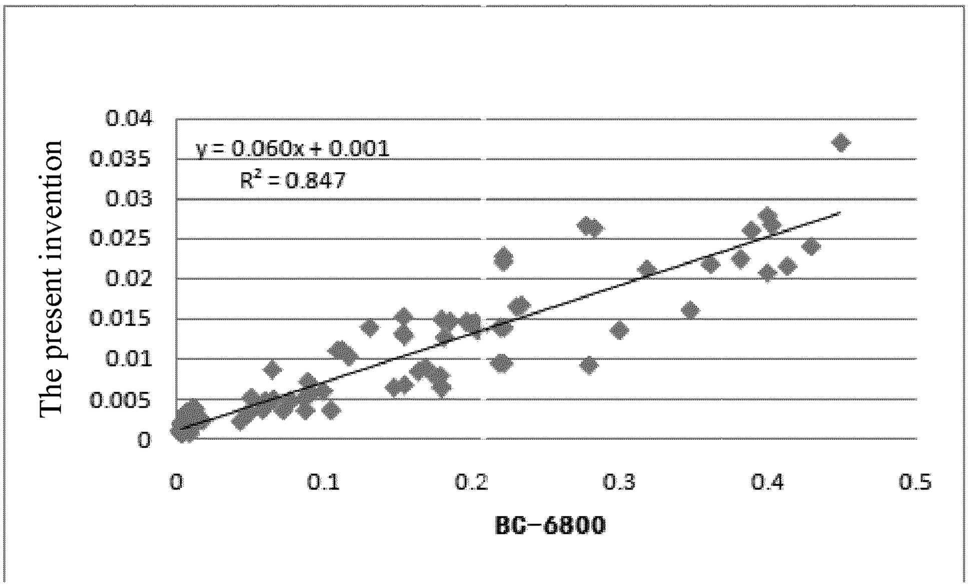

0.04
0.035
0.03
0.025
0.02
0.015
0.01
0.005
0
The present invention
y = 0.060x + 0.001
R² = 0.847
0
0.1
0.2
0.3
0.4
0.5
BC-6800


FIG. 7e

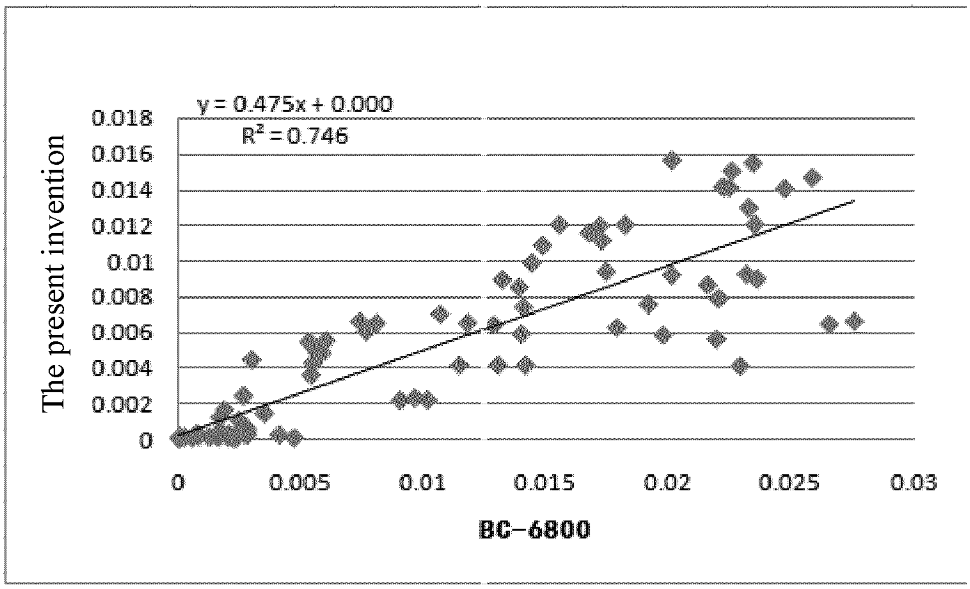

0.018
0.016
0.014
0.012
0.01
0.008
0.006
0.004
0.002
0
The present invention
y = 0.475x + 0.000
R² = 0.746
0
0.005
0.01
0.015
0.02
0.025
0.03
BC-6800


FIG. 7f

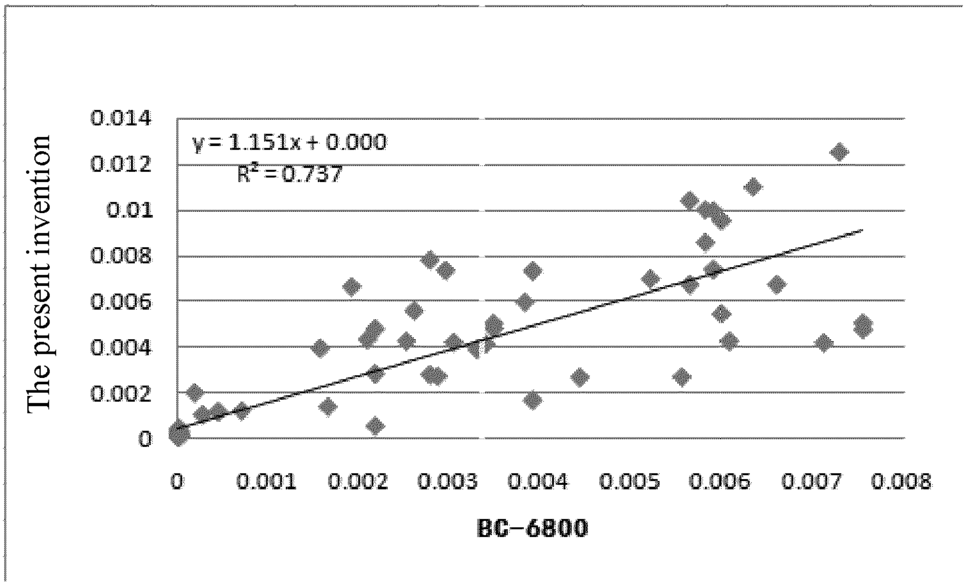
y = 1.151x + 0.000
$R^2$ = 0.737
The present invention
BC-6800
0.014
0.012
0.01
0.008
0.006
0.004
0.002
0
0
0.001
0.002
0.003
0.004
0.005
0.006
0.007
0.008

FIG. 7g

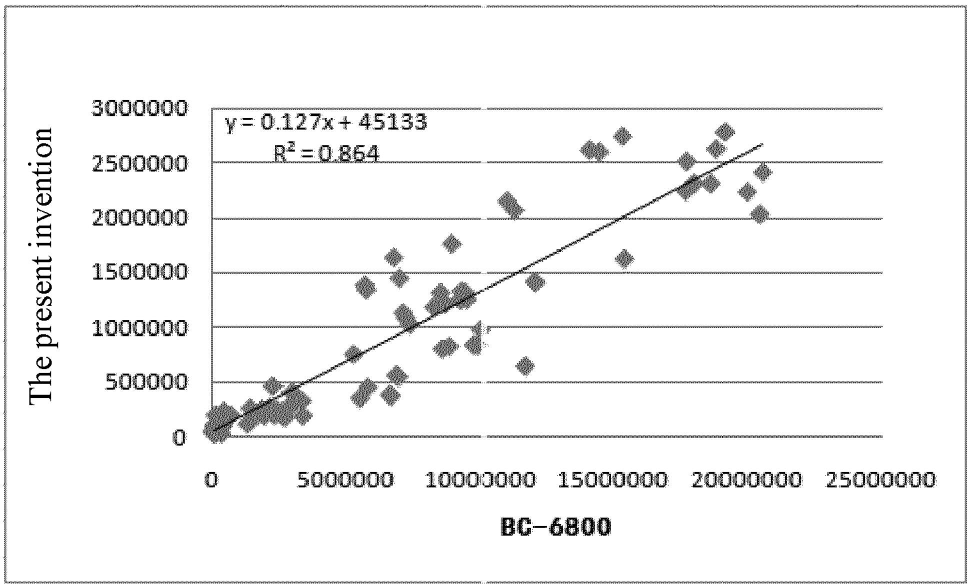
y = 0.127x + 45133
$R^2$ = 0.864
The present invention
BC-6800
3000000
2500000
2000000
1500000
1000000
500000
0
0
5000000
10000000
15000000
20000000
25000000

FIG. 7h

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 97110727
- EP 1574839 A1
- CN 101236195 A
- JP 2011237462 A
- EP 0774113 A1
- CN 104749144 A
- CN 200910177186
- CN 200910238927


### Non-patent literature cited in the description


- Forward Light Scattering Of Red Blood Cells. *Laser Biology*, June 1996, vol. 5
- **WEI ZHANG** ; **YUAN LU** ; **SHIMING DU**. Mie Scattering Characteristic Analysis Of Spherical Particles. *Optical Technology*, November 2010, vol. 36 (6), 936-939